# EUROPEAN PATENT SPECIFICATION

(11) **EP 4 313 234 B1**
(45) Date of publication and mention of the grant of the patent: **15.04.2026**
(21) Application number: 22776202.8
(22) Date of filing: 23.03.2022
(51) Int. Cl.: A61M 16/10, A61M 16/16

(54) **REMOVEABLE RESPIRATORY HUMIDIFYING DEVICE MODULE**
ENTFERNBARES ATEMBEFEUCHTUNGSVORRICHTUNGSMODUL
MODULE DE DISPOSITIF D'HUMIDIFICATION RESPIRATOIRE AMOVIBLE

(30) Priority: 23.03.2021 AU 2021900849; 23.03.2021 AU 2021900851
(43) Date of publication of application: 07.02.2024
(73) Proprietor: AUT Ventures Limited, Auckland 1010 (NZ)
(72) Inventor: AL-JUMAILY, Ahmed, Auckland, 2576 (NZ); GRAU-BARTUAL, Sandra, Auckland, 1024 (NZ); FIELDING, Michael, Devonport Auckland, 0624 (NZ); CUNNINGHAM, Helen Jean, Wellington Auckland, 1062 (NZ)
(74) Representative: ip21 Ltd
(86) International application number: PCT/NZ2022/050034
(87) International publication number: WO 2022/203523

(56) References cited:
- EP-A1- 1 671 668
- WO-A1-2019/093910
- WO-A1-2020/097673
- WO-A1-94/26339
- US-A- 5 109 471
- US-A- 5 367 604
- US-A- 5 383 447
- US-A1- 2011 259 327
- US-A1- 2012 017 905
- US-A1- 2012 017 905
- US-A1- 2017 182 280
- US-A1- 2020 164 172
- US-B2- 10 688 272
- US-B2- 7 322 566
- AL ASHRY HAITHAM S. ET AL: "Humidification during Mechanical Ventilation in the Adult Patient", BIOMED RESEARCH INTERNATIONAL, vol. 2014, 25 June 2014 (2014-06-25), pages 1 - 12, XP055870906, ISSN: 2314-6133, DOI: 10.1155/2014/715434

## Description

### FIELD

This invention relates to a removeable respiratory humidifying device module comprising a moisture exchanger.

### BACKGROUND

Respiratory support devices, such as asthma nebulizers, positive airway pressure devices (including PAP, CPAP, APAP and BiPAP), anaesthesia machines, ventilators or even tracheotomy, are used to restore or provide a proper respiration cycle in some patients. These therapies disturb the natural lubrication and normal air conditioning process because there is an increase in pressure and turbulent effect, or the nasal cavity, which is the most important air conditioning part, is bypassed. Breathing devices, can cause drying and trauma of the mucosa where ciliated cells are inactivated and reduced (Malik & Kenyon, 2004). Therefore, the inspiratory airflow has to be warmed and humidified in order to reach the lungs at 37°C with near 100% relative humidity, and to maintain an optimum mucociliary transport.

This airflow is delivered to a patient (or, in some cases, a user who is not sick) through a patient interface such as a facial mask, nasal mask, nasal pillow mask, tracheotomy tube, endotracheal tube or fabric face mask. Between the respiratory support device and the patient interface it is common to have tubes, valves, Y-connections, suction ports, sampling ports etc., together known as a breathing circuit. The type of patient interface and breathing circuit depends on the application.

WO 2019/093910 discloses a moisture exchanger that is heated to change from being hydrophilic to hydrophobic (or vice versa), and a respiratory humidifying device comprising the moisture exchanger and heating element. The respiratory humidifying device may receive supplied airflow from a respiratory support device, or it may heat and humidify ambient air.

The moisture exchanger of the respiratory humidifying device comprises a temperature-responsive polymer and a substrate that physically supports the temperature-responsive polymer to hold a desired shape and provides a large multiplicity of surfaces for the polymer to be placed in contact with the inspiratory and expiratory airflow. Examples of substrates which might be suitable include fibrous material such as natural fibres (Cotton, Linen, Chitin or Chitosan among others), synthetic or processed fibres (Rayon, Polyvinyl alcohol (PVA), or Polypropylene (PP) among others) or a mix thereof.

The temperature-responsive polymer may be grafted onto the substrate. WO 2019/093910 discloses suitable temperature-responsive polymers.

It is acknowledged that the terms "comprise", "comprises" and "comprising" may, under varying jurisdictions, be attributed with either an exclusive or an inclusive meaning. For the purpose of this specification, and unless otherwise noted, these terms are intended to have an inclusive meaning - i.e., they will be taken to mean an inclusion of the listed components which the use directly references, and possibly also of other non-specified components or elements.

The term airflow is used generally, and may include flows of oxygen-enriched air, anaesthetic gases, or pure oxygen, for example.

EP1671668 discloses a respiratory humidifier in which a housing contains a foam or paper HME material. A heater is provided outside the housing to maintain a relatively stable temperature.

US2012/017905 discloses another device using a traditional HME material such as paper or another absorbent material. Again, a heater is used to maintain a stable temperature.

WO94/26339 discloses another paper-based HME material and proposes an arrangement to reduce condensation in the device.

US7322566 discloses a passive HME material, which may be PTFE or paper.

US5109471 and US5383447 are related documents from the same inventor, both disclosing further examples of using traditional HME materials.

Al Ashry et al ("Humidification during Mechanical Ventilation in the Adult Patient", Haitham S. Al Ashry1 and Ariel M. Modrykamien, BioMed Research International Volume 2014, Article ID 715434, http://dx.doi.org/10.1155/2014/715434) discusses HME systems in general.

US5367604 relates to a "vapour transfer means" for transferring vapour but not liquid to a gas flow (which is a different mechanism to moisture exchange). A microporous material is used to form a liquid compartment. The liquid is heated and resulting vapour exits through the microporous material.

Reference to any document in this specification does not constitute an admission that it is prior art, validly combinable with other documents or that it forms part of the common general knowledge.

An object of the invention is to develop an improved respiratory humidifying device which is hygienic to use, or to at least provide the public or industry with a useful choice.

### SUMMARY OF THE INVENTION

The present invention provides a respiratory humidifying device as defined in claim 1. Further preferred embodiments of the invention are defined in the dependent claims.

### SUMMARY OF THE DISCLOSURE

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

According to one example embodiment there is provided a respiratory humidifying device for use in a respiratory humidifying system having an inspiration and expiration respiratory cycle, the respiratory humidifying device comprising:
a moisture exchanger positioned such that airflow occurring in the inspiration and expiration respiratory cycle is in contact with the moisture exchanger, the moisture exchanger being temperature-responsive and having a critical solution temperature;
a heater; and
a controller controlling a supply of power to the heater such that:
   during expiration the moisture exchanger extracts moisture from the airflow; and
   during inspiration the moisture exchanger returns moisture to the airflow,
characterised in that:
   the moisture exchanger and the heater are contained in a removeable module and where the removeable module comprises a connector which impermanently engages with a module connector configured to pass electrical signals between the controller and the removable module;
   the removeable module comprises an open upstream end for making a detachable connection for airflow from a respiratory support device or an upstream breathing circuit connected to the respiratory support device;
   and the removeable module comprises an open downstream end for making a detachable connection for airflow to and from a patient interface or a downstream breathing circuit connected to the patient interface;
   the module connector comprises the controller.

Preferably the respiratory humidifying device further comprises at least one sensor for measuring a parameter of the airflow.

Preferably the airflow parameter is or relates to the direction of the airflow.

Preferably at least one of the at least one sensor is selected from the group consisting of a temperature sensor, an airflow speed sensor, an airflow direction sensor, a pressure sensor, a CO2 sensor, a O2 sensor, a CO sensor and a humidity sensor.

Preferably the heater causes the moisture exchanger temperature to rise.

Preferably the controller supplies power to the heater in response to a measured airflow parameter.

Preferably the respiratory humidifying device further comprises a power supply for supplying power to the controller and/or the heater.

Preferably the power supply is selected from the group consisting of a battery, a DC-DC power supply and a mains (AC-DC) power supply.

Preferably the power supply and the controller are separate from the removeable module.

Preferably the removeable module comprises an open upstream end for making a detachable connection for airflow from a respiratory support device or an upstream breathing circuit connected to the respiratory support device.

Preferably the removeable module comprises an open downstream end for making a detachable connection for airflow to and from a patient interface or a downstream breathing circuit connected to the patient interface.

Preferably the only part of the respiratory humidifying device in contact with the airflow is the removeable module and the parts contained therein.

Preferably the removeable module comprises the heater.

Preferably the removeable module comprises at least one removeable module sensor.

Preferably at least one of the at least one removeable module sensor measures a parameter of the airflow or the moisture exchanger temperature without being in contact with the airflow.

Preferably at least one of the at least one removeable module sensor measures a parameter of the airflow or the moisture exchanger temperature through a wall of the removeable module.

Preferably at least one of the at least one removeable module sensor is selected from the group consisting of an ultrasonic airflow speed, a direction sensor and an infrared temperature sensor.

Preferably the removeable module comprises a viral or bacterial filter.

Preferably the viral or bacterial filter is located upstream of the moisture exchanger.

Preferably the removeable module comprises standard connectors at downstream and upstream ends for interfacing with at least one of a patient interface, a respiratory support device, a Y-connector, or a breathing circuit
Preferably the removeable module comprises a Y-connector located upstream from the moisture exchanger for connection to a respiratory support device in a dual limb configuration.

Preferably the removeable module comprises a bias vent, preferably located between 1 mm and 20 mm upstream of the moisture exchanger.

Preferably the removeable module comprises a module connector which impermanently engages with the removeable module.

Preferably the module connector passes electrical signals between the controller and the removeable module.

Preferably the module connector comprises alignment means to align the module connector with the removeable module.

Preferably the module connector is radially engageable and disengageable with the removeable module.

Preferably the module connector comprises the controller.

Preferably the module connector comprises at least one module connector sensor.

Preferably at least one of the at least one removeable module sensor measures a parameter of the airflow or the moisture exchanger temperature without being in contact with the airflow.

Preferably at least one of the at least one module connector sensor measures a parameter of the airflow or the moisture exchanger temperature through a wall of the removeable module.

Preferably at least one of the at least one module connector sensor is selected from the group consisting of an ultrasonic airflow speed, a direction sensor and an infrared temperature sensor.

Preferably the module connector comprises the heater.

Preferably the module connector comprises a user interface.

Preferably the user interface is usable for one or more of checking settings, checking operation, setting settings, or turning on and off the respiratory humidifying device.

Preferably the respiratory humidifying device further includes a user interface for one or more of checking settings, checking operation, setting settings, or turning on and off the respiratory humidifying device.

Preferably the heater is selected from the group consisting of a resistive element, a filament, a spiral wire, an inductive coil, radiation, and an infrared heater.

Preferably the heater comprises at least one first heater in contact with the moisture exchanger and at least one second heater not in contact with the moisture exchanger, and both the first and second heaters are in contact with the airflow.

Preferably the at least one second heater is located between the moisture exchanger and the respiratory support device.

Preferably the controller controls a moisture exchanger temperature using the at least one first heater and an inspiratory air temperature using the at least one second heater.

Preferably the controller controls the power to the at least one first and at least one second heaters together.

Preferably the at least one first and at least one second heaters are connected in series.

Preferably the moisture exchanger is arranged such that substantially all the airflow passing the moisture exchanger flows through the moisture exchanger.

Preferably the moisture exchanger is arranged such that there is at least one path for airflow to pass through the removeable module such that it is in contact with the moisture exchanger but does not pass through the moisture exchanger.

Preferably the moisture exchanger is arranged at a non-perpendicular angle to the airflow through the removeable module.

Preferably the moisture exchanger is of a pleated or folded construction.

Preferably the controller includes a mode selector for selecting one of a plurality of operating modes, each of the operating modes providing different amounts of, or timing of, power to the heater, in response to the measured airflow.

Preferably the mode selector comprises module sensing means to measure a physical characteristic of the removeable module, a selection table (memory) of physical characteristics of a plurality of different removeable module types and desired operation modes of the plurality of different removeable module types and selects from the selection table the operating mode that best matches the measured physical characteristic.

Preferably the module sensing means is selected from the group consisting of resistance measurement, heater resistance measurement, pinout detection, and optical sensing.

Preferably the controller comprises calibration means to measure a physical characteristic of the removeable module, and the controller is operable to detect connection of a new removeable module and to use the calibration means to measure the physical characteristic of the new removeable module.

Preferably the calibration means calibrates the heater resistance at the current temperature.

Preferably the calibration means calibrates the airflow sensor characteristics.

According to another example embodiment there is provided a removeable respiratory humidifying device module, the module configured to be positioned in the respiratory humidifying device such that at least some airflow occurring in an inspiration and expiration respiratory cycle of the respiratory humidifying device passes through the module, the module comprising a moisture exchanger, the moisture exchanger being temperature-responsive and having a critical solution temperature, wherein during expiration the moisture exchanger extracts moisture from the airflow and during inspiration the moisture exchanger returns moisture to the airflow.

Preferably the removeable respiratory humidifying device module further comprises at least one sensor.

Preferably one of the at least one of the at least one sensor measures a parameter of the airflow.

Preferably the airflow parameter is or relates to the direction of the airflow.

Preferably the at least one of the at least one sensor is selected from the group consisting of a temperature sensor, an airflow speed sensor, an airflow direction sensor, a pressure sensor, aCO2 sensor, aO2 sensor, a CO sensor and a humidity sensor.

Preferably at least one of the at least one sensor measures a parameter of the airflow or the moisture exchanger temperature without being in contact with the airflow.

Preferably at least one of the at least one sensor measures a parameter of the airflow or the moisture exchanger temperature through a wall of the removeable module.

Preferably at least one of the at least one sensor is selected from the group consisting of an ultrasonic airflow speed, a direction sensor and an infrared temperature sensor.

Preferably the moisture exchanger is arranged such that substantially all the airflow passing through the removeable respiratory humidifying device module passes through the moisture exchanger.

Preferably the removeable respiratory humidifying device module is arranged such that there is at least one path for airflow to contact with the moisture.

Preferably the moisture exchanger is arranged at a non-perpendicular angle to the airflow through the removeable respiratory humidifying device module.

Preferably the moisture exchanger is of a pleated or folded construction.

Preferably the removeable module comprises an open upstream end for making a detachable connection for airflow from a respiratory support device or an upstream breathing circuit connected to the respiratory support device.

Preferably the removeable module comprises an open downstream end for making a detachable connection for airflow to and from a patient interface or a downstream breathing circuit connected to the patient interface.

Preferably the removeable module comprises a viral or bacterial filter.

Preferably the viral or bacterial filter is located upstream of the moisture exchanger.

Preferably the removeable module comprises standard connectors at downstream and upstream ends for interfacing with at least one of a patient interface, a respiratory support device, a Y-connector, or a breathing circuit.

Preferably the removeable module comprises a Y-connector located upstream from the moisture exchanger for connection to a respiratory support device in a dual limb configuration.

Preferably the removeable module comprises a bias vent, preferably located between 1 mm and 20 mm upstream of the moisture exchanger.

Preferably the removeable module comprises a connector which impermanently engages with a module connector.

Preferably the connector comprises alignment means to align the module connector with the removeable module.

Preferably the module connector is radially engageable and disengageable with the connector.

Preferably the module connector passes electrical signals between a controller and the removeable respiratory humidifying device module.

Preferably the controller controls a supply of power to the removeable respiratory humidifying device module.

Preferably the controller further includes a user interface for one or more of checking settings, checking operation, setting settings, or turning on and off the removeable respiratory humidifying device module.

Preferably the removeable respiratory humidifying device module further comprises a heater.

Preferably the heater is selected from the group consisting of a resistive element, a filament, a spiral wire, an inductive coil, radiation, and an infrared heater.

Preferably the heater comprises at least one first heater in contact with the moisture exchanger and at least one second heater not in contact with the moisture exchanger, and both the first and second heaters are in contact with the airflow.

Preferably the at least one second heater is located between the moisture exchanger and a respiratory support device.

Preferably the controller controls a moisture exchanger temperature using the at least one first heater and an inspiratory air temperature using the at least one second heater.

Preferably the controller controls the power to the at least one first and at least one second heaters together.

Preferably the at least one first and at least one second heaters are connected in series.

Preferably the heater causes the moisture exchanger temperature to rise.

Preferably the controller supplies power to the heater in response to a measured airflow parameter.

Preferably the controller includes a mode selector for selecting one of a plurality of operating modes, each of the operating modes providing different amounts of, or timing of, power to the heater, in response to the measured airflow.

### BRIEF DESCRIPTION OF THE DRAWINGS

The accompanying drawings which are incorporated in and constitute part of the specification, illustrate embodiments of the present disclosure and, together with the general description of the disclosure given above, and the detailed description of embodiments given below, serve to explain the principles of the present disclosure, in which:
Figure 1 shows an example respiratory mask of the prior art.
Figure 2a shows an example respiratory humidifying device used with a single limb breathing circuit.
Figure 2b shows a schematic of a respiratory humidifying device used with a dual limb breathing circuit.
Figure 3a shows an example respiratory humidifying device located between an endotracheal tube and the main breathing circuit.
Figure 3b shows an example respiratory humidifying device located between nasal pillows and the main breathing circuit.
Figure 4a shows an example moisture exchanger arranged perpendicular to the air flow.
Figure 4b shows a further example moisture exchanger arranged perpendicular to the air flow, and a heating element.
Figure 4c shows an example moisture exchanger arrange at an angle to the airflow, and a heating element.
Figure 5a shows an example moisture exchanger of pleated or folded construction in line or parallel to the air flow.
Figure 5b shows an example moisture exchanger of pleated or folded construction in line or parallel to the air flow.
Figure 5c shows an example moisture exchanger of pleated or folded construction folded into a cone shape.
Figure 6 shows a bias flow vent for the respiratory humidifying device containing bias flow guides and bias flow baffles.
Figure 7 shows an example respiratory humidifying device with a removeable module located between a full faced mask and a single limb breathing circuit.
Figure 8 shows an example removeable module made from two axially-engaged parts, including a bacterial and/or viral filter and a plurality of bias vent outlets.
Figure 9a shows an example heater and flow sensor for the removeable module in Figure 8.
Figure 9b shows an example retaining means for the moisture exchanger for use with the heater and flow sensor in Figure 9b.
Figure 10 shows several example respiratory humidifying devices with separate power supplies.
Figure 11 shows several example respiratory humidifying devices with wall-mounted power supplies.
Figure 12 shows an example user interface for a respiratory humidifying device.
Figure 13 shows example respiratory humidifying device in which the controller is designed to hang and be used with a module connector and a removeable module.
Figure 14 shows an example respiratory humidifying device in which the controller is designed to be placed on a patient's bed and be used with a module connector and a removeable module.
Figure 15 shows an example respiratory humidifying device in which the controller and the module connector are integrated and also comprise a touch screen user interface.
Figure 16a shows an exploded view of a removeable module.
Figure 16b shows an example sensor arrangement inside the removeable module of Figure 16a.
Figure 16c shows an example connecting cable between a removeable module and a controller.
Figures 17a-e shows various aspects of an example removeable module and module connector which includes sensors.
Figure 18 shows the parts of the respiratory humidifying device of Figure 17a, but with one less temperature sensor.
Figure 19 shows part of an example respiratory humidifying device with a removeable module and with electronics integrated into a module connector.
Figure 20 shows a selection table in the memory of a controller.
Figure 21 shows a flow diagram of an algorithm for calibrating the heater resistance.
Figure 22a shows the respiratory humidifying device in use with a single limb breathing circuit and exhaust valve.
Figure 22b shows the respiratory humidifying device in use with an endotracheal tube with dual limb breathing circuit and Positive End Expiratory Pressure (PEEP) clinical arrangement.
Figure 22c shows the respiratory humidifying device in use with a non-invasive ventilation (NIV) mask with single limb and exhaust valve breathing circuit.
Figure 22d shows the respiratory humidifying device in use with a non-invasive ventilation (NIV) mask with a dual limb and PEEP valve breathing circuit.
Figure 23a illustrates a typical airflow and corresponding controller output during breathing of the prior art device.
Figure 23b illustrates a typical airflow and corresponding controller output during breathing of the respiratory humidifying device of the current invention.

### DETAILED DESCRIPTION

### Arrangement

### General arrangement & bias venting

The respiratory humidifying device can be integrated in the patient interface, such as CPAP and Non-invasive Ventilation (NIV) masks, where the moisture exchanger is located inside the mask; or located between the mask frame inlet and the main breathing circuit.

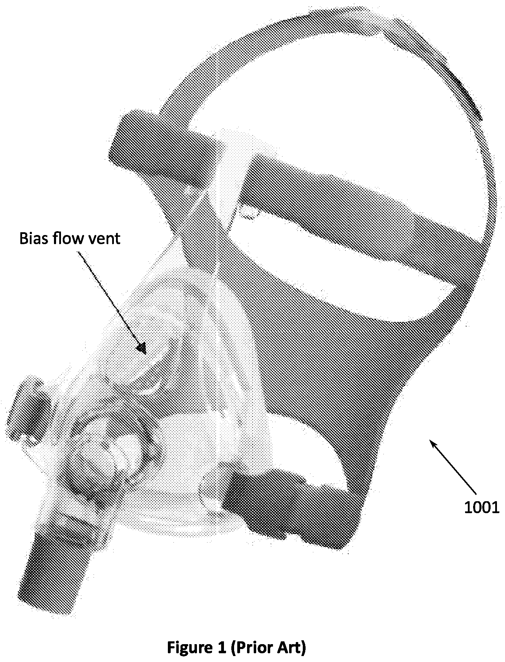

Many CPAP and Non-invasive Ventilation (NIV) patient interfaces have a bias flow vent (outlet) located on the mask frame or elbow (see Figure 1, prior art, Fisher & Paykel Simplus 1001 full face mask with bias flow vent holes located on the upper region of the mask frame, above the elbow). The bias flow vent lets a continuous, potentially varying, flow of air leave the breathing circuit, including substantially all the air expired by the patient.

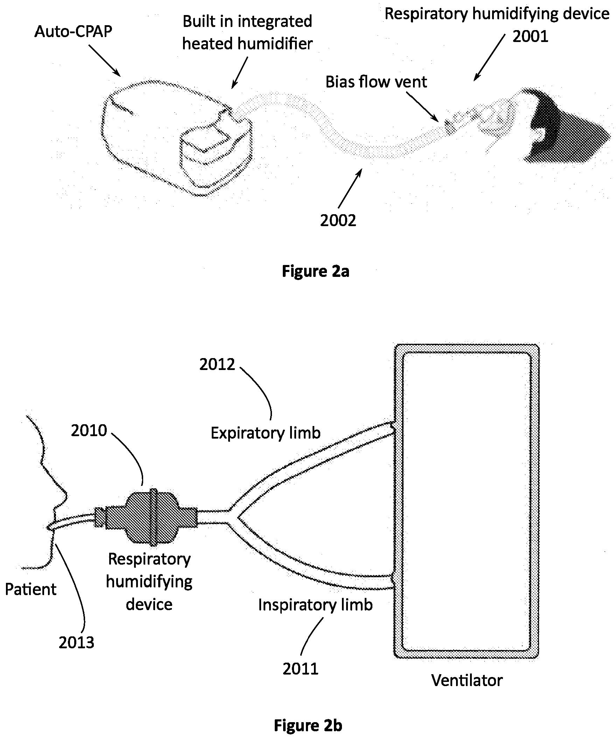

Figure 2a shows that a respiratory humidifying device 2001 of the invention, or parts thereof (for example the heater, moisture exchanger and/or user interface), is preferably located in a self-contained unit, similar in size to a prior art HME, connected in a breathing circuit between the air delivery tube and the patient interface. The respiratory humidifying device could even be incorporated into the breathing circuit, for example as part of the main breathing tube, so it is replaced as part of the breathing circuit.

Figure 2a shows a single limb breathing circuit 2002. The bias flow vent is located on the upstream or non-patient side (the respiratory support device side) of the moisture exchanger. Patient interfaces (e.g. masks) with no bias flow vent on the frame or elbow (non-vented patient interface masks) need to be used. This bias flow vent location directs all the expired air (from the patient) through the moisture exchanger, allowing for recapture of the patient's moisture.

Figure 2b shows a schematic of the respiratory humidifying device 2010 used in a dual limb breathing circuit. In this case, there is no bias flow vent in the patient interface, respiratory humidifying device nor breathing circuit, as the ventilator (respiratory support device) receives the expired air and is responsible for exhausting or recirculating it. The respiratory humidifying device would be located on the patient leg of the Y junction connecting the inspiratory limb 2011, the expiratory limb 2012 and the patient interface 2013, close to the junction. The respiratory humidifying device may even incorporate the Y junction and may therefore have two upstream connectors and one downstream connector.

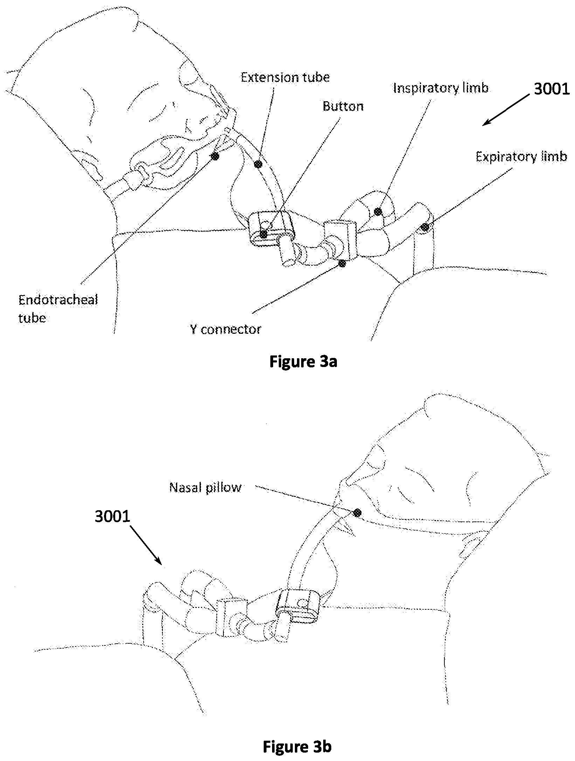

Figure 3a and Figure 3b also show a respiratory humidifying device 3001 used in a dual limb breathing circuit. This respiratory humidifying device includes a user interface comprising a button, and incorporates within it a battery as the power supply (not shown). The respiratory humidifying device 3001 may also include an extension tube to connect to an endotracheal tube (Figure 3a) or nasal pillow (Figure 3b) patient interface on the downstream end. On the upstream end, the breathing circuit may comprise an inspiratory limb and an expiratory limb, connected to the respiratory humidifying device via a Y-connector.

### Connectors

Masks designed with no bias flow vent on the frame, elbow or generally on the patient side of the respiratory humidifying device should have mechanical connections that are designed not to connect directly to standard CPAP circuits, such as 22mm tapered connectors (or similar ISO standard connectors). This will prevent the patient or healthcare providers from accidently connecting standard CPAP breathing circuits directly to the mask, without using the respiratory humidifying device. Doing so may harm the patient as no bias flow vent would be provided in the system (of a non-vented mask). Warnings, colours and/or words may be placed on such masks to provide additional protection.

The connector on the non-patient (upstream) side of the respiratory humidifying device may be any standard ISO medical connector, such as a 22mm male or female tapered connector. This will enable the respiratory humidifying device to connect to standard hospital or home breathing circuits. There may be more than one upstream connector.

The patient (downstream) side of the respiratory humidifying device preferably should not have a standard removeable connector, in order to avoid accidentally connecting it to a non-vented mask, when the respiratory humidifying device is designed to be used with a non-vented mask. Alternatively, the downstream connector may be a standard connector, depending on the application.

### Components

Examples of substrates which might be suitable include fibrous material such as natural fibres (Cotton, Linen, Chitin or Chitosan among others), synthetic or processed fibres (Rayon, Polyvinyl alcohol (PVA), or Polypropylene (PP) among others) or a mix thereof, and non-fibrous materials with a very large number of grains, crystals or pores such as certain manufactured or naturally occurring structures of metals or polymers. Substrates may be created with a regular structure or an irregular structure or a combination of both, for example a woven textile or a non-woven textile, a 3D printed metal matrix or a sintered metal matrix. Some implementations may include a substrate which is a textile while others may use an open-celled foam or yet other suitable materials.

The necessary characteristics of substrates suitable for use with the invention include that they have a very large surface area compared to their volume, and permit air to pass through easily. Ideally the substrate is porous, with a porosity exceeding 10% or 25%, and more preferably 50% or 75%.

The temperature-responsive polymer may be grafted onto the substrate; a co-polymer grafted from the substrate; polymerized to form the substrate; or a co-polymer polymerized to form the substrate. The substrate and temperature-responsive polymer may be the same, for example: a network polymer or co-polymer structure; a network polymeric structure made from one or more polymers or co-polymers; electro-spun fibres; woven fibres; or non-woven fibres.

### Moisture exchanger arrangements

The respiratory humidifying device requires a moisture exchanger to be positioned in the airflow.

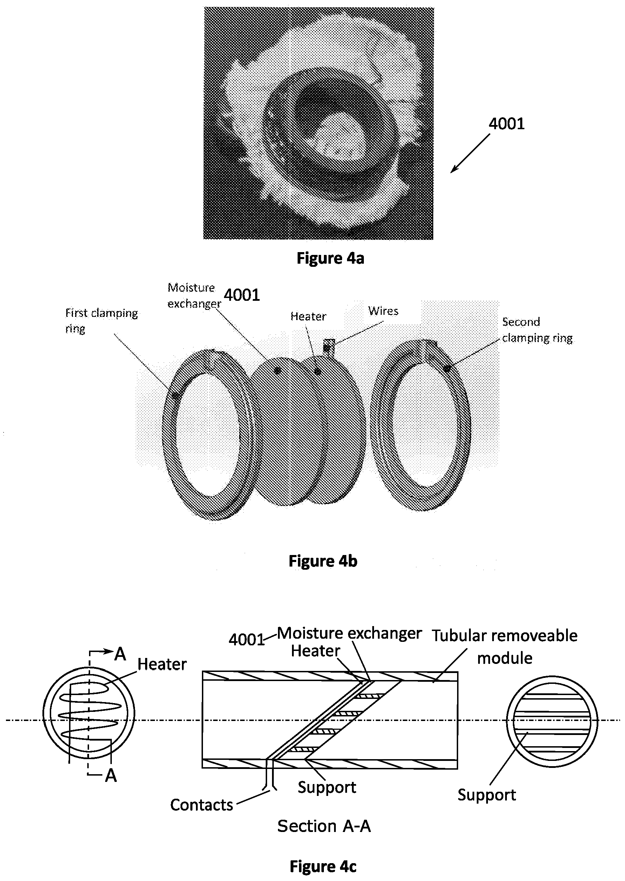

Figure 4a and Figure 4b show a moisture exchanger 4001 which is placed perpendicular to the airflow, such that all or substantially all the airflow must pass through the substrate of the moisture exchanger during expiration or inspiration. The moisture exchanger is held against a disc-shaped and substantially porous or open heater (shown here in schematic form) by first and second clamping rings. Raised edges of the second clamping ring fix the heater aligned with the central axis, and wires carry power to the heater from a controller. The moisture exchanger is cut in a disc shape which fits concentrically in raised edges of the first clamping ring to fix the moisture exchanger aligned with the central axis. The moisture exchanger may be two or more layers of substrate and temperature-responsive polymer, to increase the water retaining properties and therefore the performance of the respiratory humidifying device.

Figure 4c shows a tubular removeable module containing a moisture exchanger 4001 which lies at an angle to the axis of the tube. The moisture exchanger area is therefore increased relative to the cross-sectional area of the removeable module, allowing a larger moisture exchanger to capture more moisture while introducing less pressure drop across the moisture exchanger when airflow passes from one end of the tube to the other. A support in the form of a series of horizontal bars lies diagonally across the tube, against which a heater of criss-crossing bent wire holds the moisture exchanger. The heater wires pass through the side of the tube to contacts on the outside. All the airflow must pass through the substrate of the moisture exchanger during expiration or inspiration.

### Pleated and folded moisture exchangers

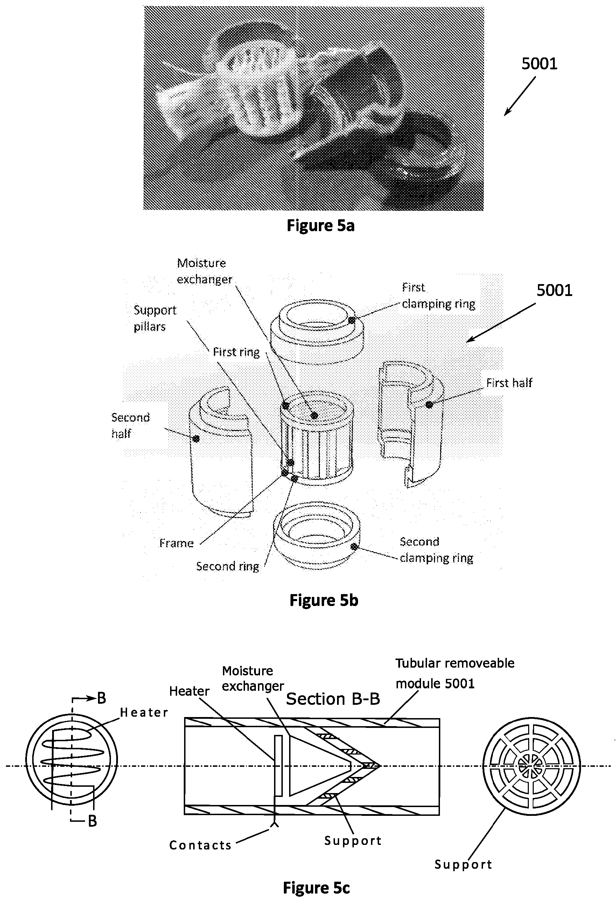

Figure 5a and Figure 5b show another tubular removeable module 5001 containing a moisture exchanger of pleated or folded construction which is parallel to but in contact with the airflow, such that airflow contacts the moisture exchanger but does not necessarily pass through the substrate during expiration or inspiration. The removeable module encloses the moisture exchanger in first and second half shells which are held together and around the moisture exchanger by first and second clamping rings. Any other known method for joining plastic housings (half shells), such as gluing, welding, snap fit connections etc. could be used. A frame is formed from two parallel rings connected by several support pillars, around which is folded the moisture exchanger so that it criss-crosses the airflow path between the two rings. The moisture exchanger has heater elements sewn into it. In use, the removeable module is placed in the respiratory airflow path such that the airflow passes through the centres of the first and second clamping rings, and over the surfaces of the folded moisture exchanger.

Figure 5c shows yet another tubular removeable module 5001, this one having the moisture exchanger folded into a cone shape with its axis coincident with the axis of the tube. The moisture exchanger area is therefore increased relative to the cross-sectional area of the removeable module, allowing a larger moisture exchanger to capture more moisture while introducing less pressure drop across the moisture exchanger when airflow passes from one end of the tube to the other. A substantially open conical support is formed inside the tube, against which a heater of crisscrossing (or spiral) bent wire holds the moisture exchanger. The heater wires pass through the side of the tube to contacts on the outside. All the airflow must pass through the substrate of the moisture exchanger during expiration or inspiration.

### Integral bias vent & design

In some applications, for example CPAP, APAP or BiPAP shown in Figure 2a, it is preferable that the respiratory humidifying device incorporates an integral bias flow vent located upstream of the moisture exchanger i.e. on the respiratory support device or non-patient side.

The bias flow vent may be incorporated into the respiratory humidifying device or removeable module housing and may be a single or multiple holes and may additionally be covered with a diffuser or filter-like material to diffuse the flow of air escaping out the bias flow vent to reduce patient annoyance.

Preferably the bias flow vent is located close to the moisture exchanger, preferably within 5 cm or more preferably within 3 cm of the moisture exchanger, and is positioned such that air supplied by a respiratory support device reaches the bias flow vent before reaching the moisture exchanger.

Preferably the bias flow vent is arranged such that some of the bias flow passing therethrough is directed against the moisture exchanger to create a cooling flow, which cools the moisture exchanger during expiration or at the end of inspiration. The cooling flow may be turbulent eddies of the bias flow created by the bias flow exiting through the bias flow vent, or it may be the bias flow itself directed parallel with the surface of the moisture exchanger by virtue of the relative arrangement of an upstream connector supplying air from the respiratory support device and the bias flow vent. Therefore, there may be bias flow guides within the respiratory humidifying device which direct the cooling flow to the moisture exchanger.

The cooling flow must not be so strong as to substantially pass through the moisture exchanger, as in doing so it would cool the walls of the device downstream (towards the patient) of the moisture exchanger and thereby create condensation. Therefore, there may also be bias flow baffles on the opposite side of the moisture exchanger to the bias flow vent to prevent the cooling flow from cooling. Preferably also the bias flow vent is at least 1cm, and preferably at least 2cm, from the moisture exchanger.

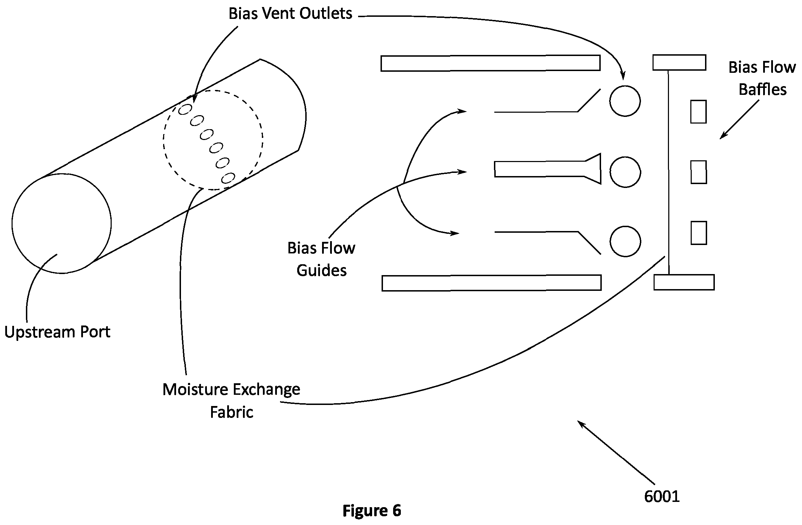

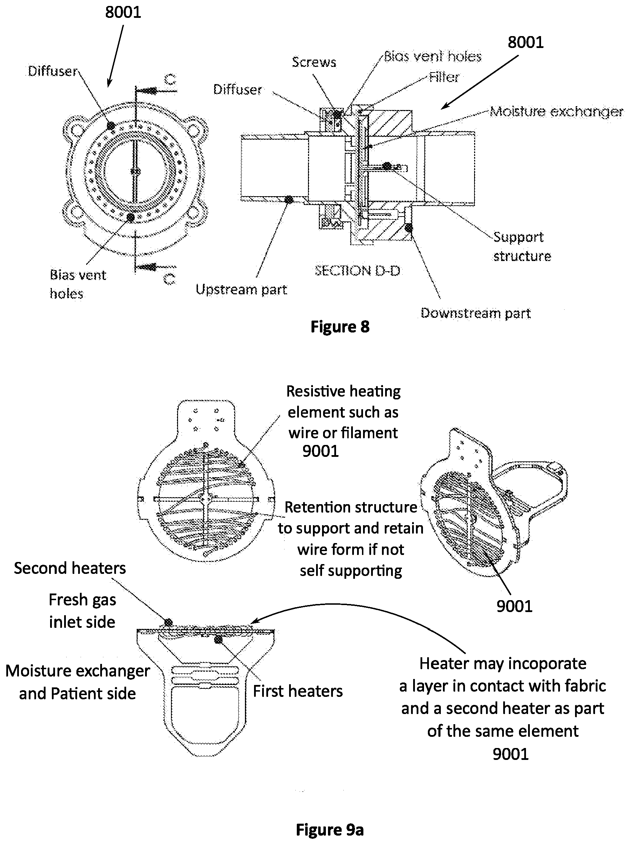

Figure 6 shows an example embodiment of part of a respiratory humidifying device 6001 with a radially arranged bias flow vent. Figure 8 also shows bias flow guides and bias flow baffles. The change of direction of the bias flow to exit the respiratory humidifying device through the bias flow vent creates eddies in the area of the moisture exchanger that serve to cool the moisture exchanger. There are further provided bias flow guides to enhance the cooling effect of those eddies by directing them against the moisture exchanger, and bias flow baffles to reduce the penetration of the eddies through the moisture exchanger which would cool the downstream components and create undesirable condensation.

### Removeable module

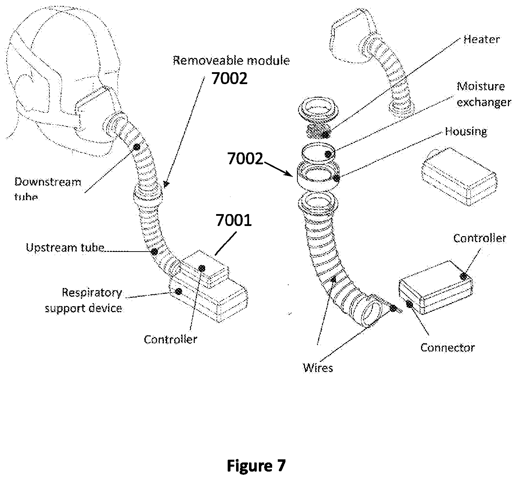

Figure 7 shows a respiratory humidifying device 7001 in which the moisture exchanger and heater are contained within a removeable module 7002, able to be removed from the breathing circuit and replaced when required. While Figure 7 shows the removeable module in a single limb breathing circuit and using a full-face mask, the removeable module is equally useful with dual limb breathing circuits and with other patient interfaces such as nasal pillow masks and endotracheal tubes.

The removeable module comprises a moisture exchanger as shown in Figure 4 and a heater held concentrically inside a circular housing. Upstream and downstream tubes rotationally latch into the housing and hold the moisture exchanger and heater in place and in firm axial contact with each other. The moisture exchange shown in Figure 5 may also be used with the removeable module.

The upstream tube contains two wires to carry power from the controller to the heater. The heater is held in contact with the two wires by rotationally latching the upstream and downstream tubes into the housing, at a predetermined rotation relative to the housing and the upstream tube determined by its design and the design of the housing and arranged to ensure that the heater is powered by the two wires coming from the upstream tube.

The upstream tube connects to the respiratory support device and the two wires connect to the controller of the respiratory humidifying device via a connector, so that the controller can heat the moisture exchanger. The wires may be formed into the upstream tube, or may be removably clipped to it, or may take another path altogether.

In use, the removeable module may be separated by a twisting motion, allowing the moisture exchanger and/or heater to be replaced. Also, the removeable module may be replaced at different times to the upstream or downstream tubes.

In an alternative embodiment, the housing is part of the upstream tube, the heater is fixed in the housing, and only the moisture exchanger is removeable.

### Electronic controller

The controller, sensors and power supply of the respiratory humidifying device may all be located within one housing also containing the moisture exchanger and heater, as shown in Figure 3a and Figure 3b. Alternatively, some or all of these functions may be located distant from the moisture exchanger, with wire(s) delivering power to the heater from the distant controller as shown in Figure 7.

For example, in Figure 7, a prior art respiratory support device may already have flow sensors, microcontrollers and power supplies. These functions can be provided to the respiratory humidifying device by the respiratory support device, where the respiratory support device measures the patient breathing flow rate, sending the signals to the respiratory humidifying device controller to determine when power needs to be provided to the heater and to provide power, via the two wires to the heater. The controller of the respiratory humidifying device may be implemented in the controller of the respiratory support device, and the two devices may be integrated into one unit.

The wires may be held to the breathing circuit, mechanically clipped or secured to the outside of the breathing circuit. Alternatively, they may be located inside the breathing circuit or built into the wall of the breathing circuit, as is common with heated wall respiratory support device tubes and is shown in Figure 7. Connecting the breathing circuit to the respiratory support device and respiratory humidifying device (at the other end) may also form a connection for the electrical circuit, powering or otherwise communicating electrically/electronically with the respiratory humidifying device.

Signals may also be sent between the respiratory humidifying device housing and respiratory support device via wireless technology, such as Bluetooth, WiFi or inductive power transfer with communication.

### Bacterial/viral filter

The respiratory humidifying device, and in particular the removeable module, can include zero, one or more integral bacterial/viral filters in the inspiratory or expiratory airflow path. Where the respiratory device includes zero integral filters, it may be used with an external filter(s) fitted to the main breathing circuit to filter inspiratory or expiratory flow, or both.

Figure 8 shows a removeable module 8001 which includes an integral bacterial and/or viral filter located between the moisture exchanger and the non-patient device outlet. This removeable module further includes a bias flow vent, arranged as a multitude of axially-directed holes around the circumference of the upstream connector. A ring-shaped diffuser encircling the upstream connector and covering the bias flow vent holes reduces the noise caused by air escaping from the holes.

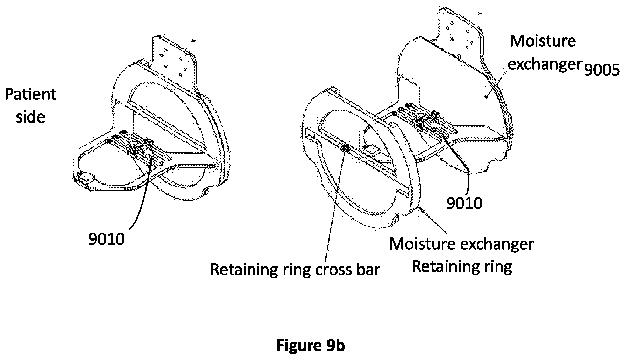

The removeable module 8001 in Figure 8 further includes a support structure comprising a heater and sensors in the airflow path (described below with reference to Figure 9), and a downstream connector for connecting to a patient interface. The moisture exchanger is held in axial engagement with the heater by the upstream part and the downstream part which are held together by screws. The removeable module includes a connector (not shown) for a cable to the controller which provides power to the heater and reads the signals from the sensors.

Where a bias flow vent is also integrated and expiratory filtering is required, the filter, or filters, should be located between the moisture exchanger and any bias flow outlet vents.

Filter embodiments may include one or more individual filters to address the functions described above. Where filtration material is directional, the more hydrophobic filtration surface shall be orientated to face the moisture exchanger for each filter. The bacterial/viral filter(s) and design embodiment of filter retention shall ensure all airflow in the required filtration directions must pass through the filter.

The respiratory humidifying device may be used in combination with a breathing system filter such as pleated hydrophobic filter or electrostatic filter placed upstream of the moisture exchanger in order to provide bacterial and viral filtration (>99%) to provide an extra protection to the patient.

### Heater

### Resistive

The heater preferably comprises a resistive heater in thermal contact (preferably direct thermal contact) with the moisture exchanger. Examples of a resistive heater include wire or conductive polymer filaments sewn into or woven into the moisture exchanger, or said filaments pressed into contact with the moisture exchanger, or a flexible printed circuit glued to or otherwise pressed against them. An exemplary heater and moisture exchanger arrangement is described below.

Figure 9a shows a resistive heating element 9001 that is threaded through holes in a support structure, and which is useable with the removeable module described with reference to Figure 8. The resistive heating element is wound to provide an even heat distribution across the moisture exchanger. The hole pattern and surrounding support structures are set within the flow path radius to maximise the exposure of the resistive heater element to the airflow and enhance cooling. Heater element support spacing is set above the minimum safe clearance to avoid electrical arcing and short circuits. The hole arrangement allows for a multitude of heater element threading patterns and heater elements that are both flexible and self-supporting. Different threading arrangements can be used to increase or decrease the length of heater element directly in contact with the moisture exchanger and the length more directly in contact with the fresh air inflow. An alternative to holes may be used, such as slots formed in the outside of the support structure that allow the heating element to be wound to the support structure to improve manufacturability. Winding the heating element around vertical posts is still another alternative option.

Figure 9b shows how the proximity of the moisture exchanger 9005 to the heater 9010 is set by a moisture exchanger retaining ring. Tension on the moisture exchanger is set by clearances between the retaining ring and the support structure. During inspiration the moisture exchanger is pulled against the heater element ensuring maximum heat transfer. During expiration the clearance between the retaining ring cross bar and the heating element may allow the moisture exchanger to reduce contact with the heater element to enhance cooling.

### Infrared

In some embodiments the heater may be an infrared heater. An infrared heater offers many advantages, such as it can almost instantaneously be turned on and/or off (there is less lag than a wire heater element). An infrared heat may be hygienically separated from the moisture exchanger and inspiratory and expiratory airflow path. This may reduce the cost of operation, as the infrared heat source could be reusable, either between the same or other patients. The infrared heat source could be sealed in a glass or plastic housing, along with the controller, or otherwise separated from the airflow by a light permeable or light bending material.

Any reflected infrared light could also be detected by a light sensor in order to calculate the humidity level of the air passing though the device (being inhaled and/or exhaled by the patient), or the moisture capture by the moisture exchanger.

### Plurality

There may be more than one heater that could be controlled together or independently to deliver different temperature profiles to different locations of the respiratory humidifying device or to different regions of the moisture exchanger.

Figure 9a shows a support structure inside a removeable module of a respiratory humidifying device. The support structure incorporates multiple first heaters and second heaters. In this example the first and second heaters are formed from one continuous piece of wire threaded through the retention structure and are therefore controlled together by the controller, but the multiple first heaters will be in contact with both the moisture exchanger and the airflow whereas the multiple second heaters are not in contact with the moisture exchanger but are still in contact with the airflow. (Figure 9b shows the location of the moisture exchanger in relation to the support structure.)

With cold room conditions, a respiratory humidifying device with just a first heater may not allow the moisture exchanger or the heater to cool down quick enough to capture substantially all of the patient's expired moisture. Therefore, a second heater which is not in contact with the moisture exchanger may cool down faster. Furthermore, during inspiration the second heater is able to pass more thermal energy to the inspired air before it reaches the moisture exchanger; with just a first heater, the first heater may have to be so hot that it would damage the moisture exchanger. Therefore, having two heaters provides a better control of the humidifying performance and more comfort to the user.

Various combination of heater and location (e.g. upstream or downstream of the moisture exchange) are possible. Multiple heaters may also be used, and they may be controlled separately or together by the controller.

### Power supplies and batteries

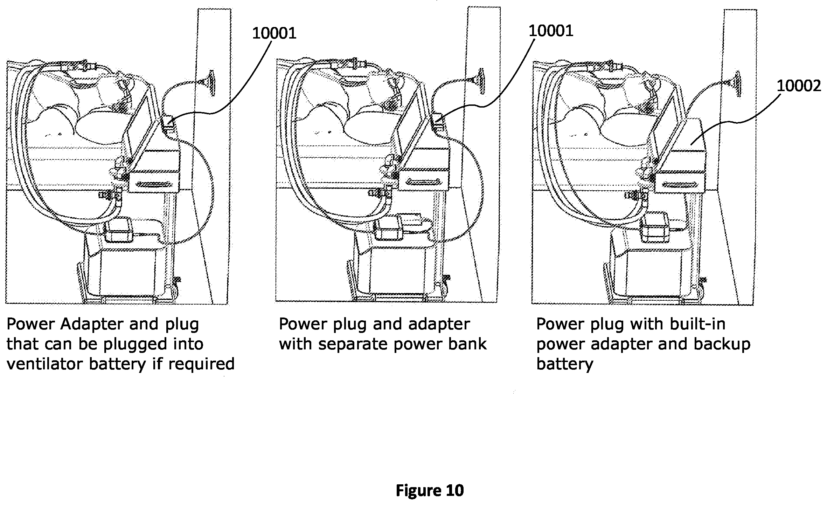

Figure 10 shows a power supply 10001 with separate plug and AC/DC adapter supplying power to the controller on its own (Figure 10a), with a separate battery pack connected in parallel or series with the controller (Figure 10b), or with a battery integrated into the controller enclosure 10002 or able to be removably attached to it to form a cohesive unit (Figure 10c).

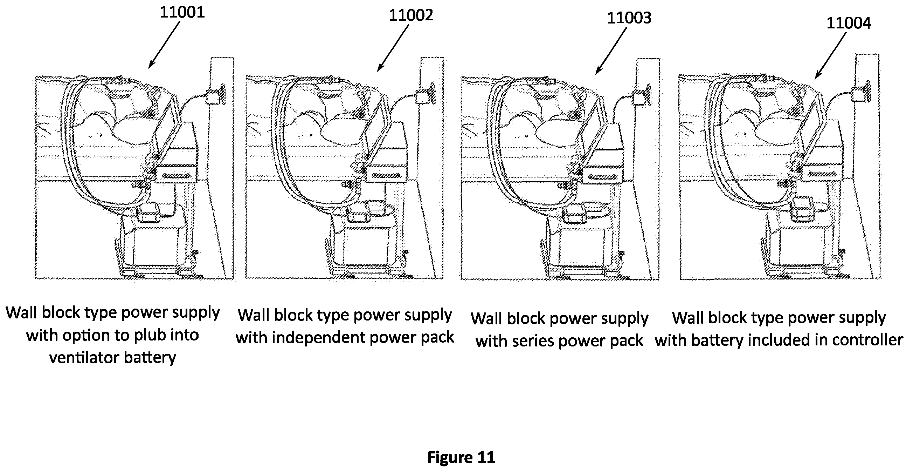

Figure 11 shows an integrated wall power plug and AC/DC adapter supplying power to the controller on its own 11001 (Figure 11a), with a separate battery pack connected in parallel with the integrated wall power plug as an alternative source of power for the controller 11002 (Figure 11b), with a separate battery pack connected in series with the integrated wall power plug as an alternative source of power for the controller 11003 (Figure 11c), or with a battery integrated into the controller enclosure or able to be removably attached to it to form a cohesive unit 11004 (Figure 11d).

### User interface - presence

The respiratory humidifying device can include a user interface for turning the respiratory humidifying device on and off, changing the humidification performance, cancelling alarms, or checking on the respiratory humidifying device operation. To achieve these goals, the user interface can change parameters related to the desired inspiratory airflow. These parameters can determine, through look up tables or calculations, the target points or target temperatures used to control the timing and magnitude of power applied to the heater.

### User interface - features

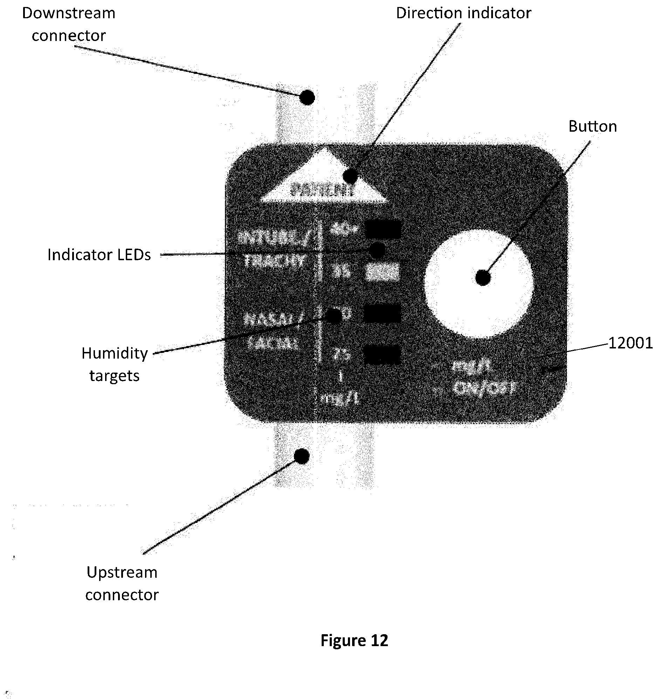

Figure 12 shows an example of the user interface 12001 integrated into a respiratory humidifying device having an upstream connector for connection to a respiratory support device, and a downstream connector for connection to a patient interface. The user interface includes four LED indicators, each next to one of four different humidity targets. Patients that are on nasal CPAP ventilation may require a relatively low level of humidification (25mg/L); patients on full face bi-level ventilation may require a higher level of humidification (30mg/L); tracheostomy patients may require 35mg/L; and intubated patients may require the highest relatively level of humidification (40mg/L or more). Pressing the button will cycle through the four different humidity targets, changing the target points and target temperatures, and the timing used by the controller. Pressing and holding the button will toggle the respiratory humidifying device on and off.

In alternative embodiments, a button may select between nasal, full face or intubated/tracheostomy options, or a knob might select a humidification or temperature anywhere within a range of 20mg/L to 40mg/L or more. The user interface may include the selection and/or display of multiple parameters, such as independently setting/displaying temperature, humidity and/or absolute or relatively humidity.

The user interface may include both an indication of the selected setting and an indication of the achieved level of performance, for example inspiratory airflow humidity or temperature. For example, a red LED indicator could show that a setting has been chosen, but not yet achieved, and a green LED indicator could show that the selected level of humidification is being delivered.

The user interface may also incorporate sounds and/or lights to provide alarms that the respiratory humidifying device has an error condition, for example due to excessive mask leak that may lower the delivered humidification level.

The respiratory humidifying device may include controls for calibrating the respiratory humidifying device or its sensors, changing the function or operation of status lights or alarms, or connect or disconnect a remote management device. Some controls may have multiple functions.

The respiratory humidifying device may incorporate status indicators such as graphical displays, value readouts, lights and speakers or beepers to indicate the functionality of the device (such as a mode or setpoint), the battery charge, correct operation, error conditions, a need to change any consumable element or removeable module, or connection to or disconnection from a remote management device. The user interface may also allow users to configure a sequence of changes in respiratory humidifying device humidification and/or temperature performance over time, e.g. 1 hour at 20mg/L humidity, 4 hours at 25mg/L. The user interface may also allow the display of recorded data on respiratory humidifying device performance or use over time periods, such as the previous hour, 24 hours, or longer periods.

### Remote management

The respiratory humidifying device may have a remote management interface to allow a remote management device to configure the respiratory humidifying device.

The remote management device preferably uses the remote management interface to achieve all the functions described above with reference to the user interface. The remote management device can preferably also retrieve data from the respiratory humidifying device, for example times and amounts of usage or non-usage; inspiratory and expiratory airflow parameters measured by sensors or otherwise inferred; occurrence or frequency of episodes of sleep apnoea, respiratory distress, difficulty breathing etc; the need for or act of replacement of parts; or error conditions.

The remote management interface may comprise one or more of wireless communication (e.g. cellular network, Wide Area Network e.g. Lorawan, Sigfox, Bluetooth, WiFi, Zigbee, Z-Wave, NFC) or wired communication (e.g. USB, l2C, SPI, RS-232, RS-485).

The remote management device may be a respiratory support device, mobile phone, tablet computer, computer server or internet cloud service. The remote management device may be an artificial intelligence remote management device which is able to retrieve data from the respiratory humidifying device and to take actions based on the data including configuring the respiratory humidifying device and alerting human users or patients.

### User interface - arrangement

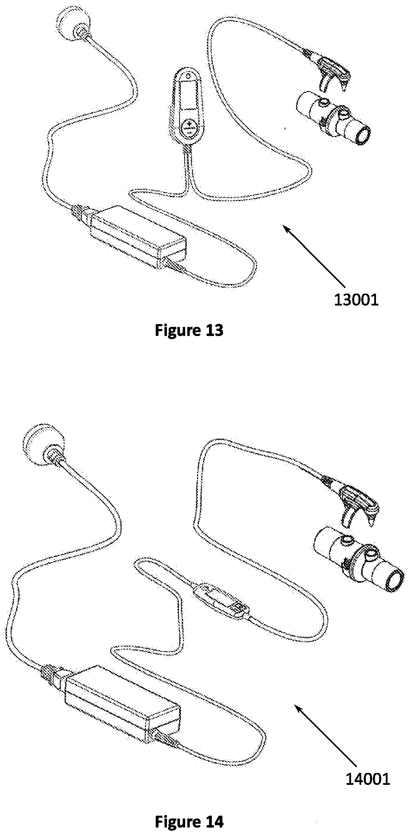

Figures 13 and 14 show example respiratory humidifying devices (13001 and 14001) in which the removeable module is separated from a controller by cable, and the cable ends in a module connector specially designed to connect to the removeable module.

Figure 13 shows a controller that can hang from an IV pole or be mounted on a ventilator or other equipment and can include any combination of exemplars previously described. The controller includes + and - buttons for changing the humidification settings, and an LCD screen for displaying the current performance and any error messages. It is connected to a module connector via a control cable.

Figure 14 shows a controller that lies or hangs inline with the control cable and can include any combination of previous exemplars previously described. The controller includes + and - buttons for changing the humidification settings, and an LCD screen for displaying the current performance and any error messages. It is connected to a module connector via a control cable.

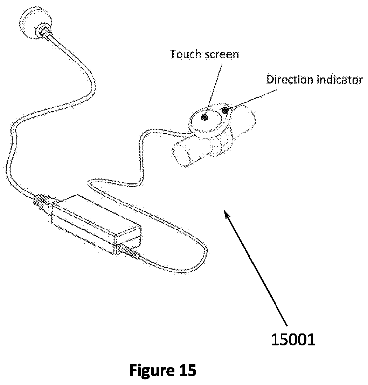

Figure 15 shows another example respiratory humidifying device 15001 in which a controller which is integrated into a module connector. The controller includes a touch screen user interface that is moulded into the cable at the module connector and a direction indicator (indicated by the pointed shape of the module connector in the example).

In each of Figures 13, 14 and 15, the controller is connected to a power supply by a power cable, and the module connector and removeable module may be separated to replace the removeable module.

The user interface and the controller do not have to be in the same unit. For example, it is also possible for the controller to be placed in the module connector, and the user interface to be a separate unit. Such an embodiment would be indistinguishable from Figure 13 or 14 from the outside.

### Mechanical controller

Rather than using an electronic controller to determine when to provide power to the heater, the controller may be a mechanical switch used to control power to the heater. For example, a mechanical switch could be located within the air flow path. The switch could be toggled from on to off by the change in direction of the patient's breath. When the patient inhales the flow switch could toggle to on, and supply power to the heater. When the patient breaths out the flow switch could be toggled to off, removing power from the heater and thus mechanically controlling power to the heater.

The mechanical flow switch is preferably biased, for example with the use of a spring, magnet or other system, to be on, or off. For example, if the flow switch is biased to be on, when the patient is nearing the end of their expiration cycle, the biasing of the flow switch will cause the switch to change to the on state, thus providing power to the heater allowing it time to heat up and provide heat to the moisture exchanger for the start of the inspiration part of the respiratory cycle. Alternatively, if the flow switch is biased to be off, when the patient is nearing the end of their inspiration cycle, the biasing of the flow switch will cause the switch to change to the off state, this removing power from the heater allowing the moisture exchanger time to cool down before the start of the expiration part of the respiratory cycle. This bias can be user adjustable to delivery different levels of humidity and/or temperature.

### Removeable module sensors

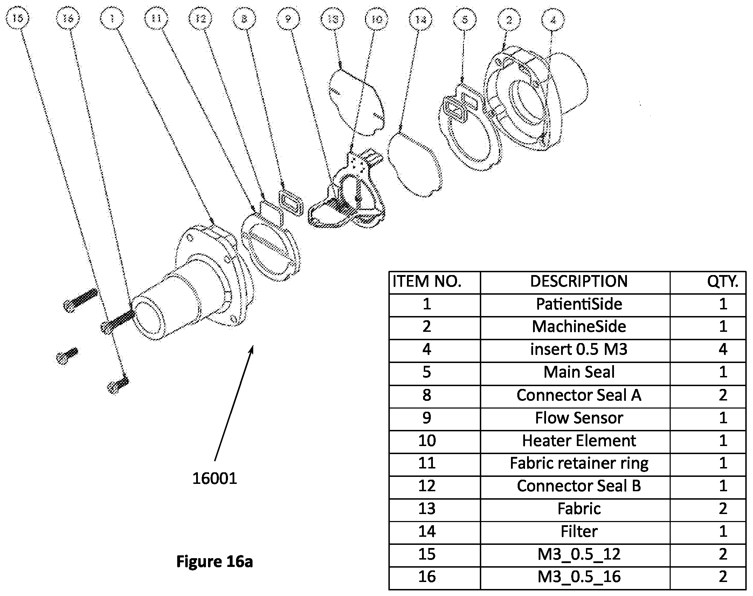

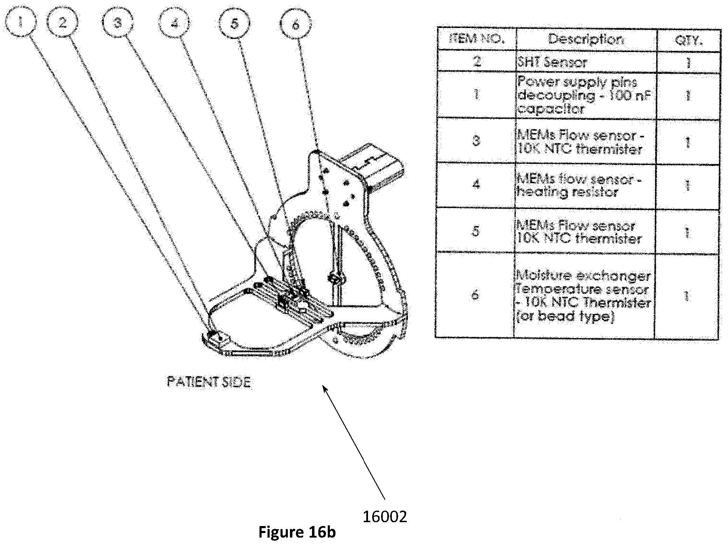

Figure 16a shows a removeable module 16001 which contains all the components of the respiratory humidifying device that are in contact with the inspiratory or expiratory airflow. Figure 16b shows detail of a support structure 16002 for the heater and sensors in Figure 16a. In this example, no parts of the respiratory humidifying device other than the removeable module are in contact with the inspiratory or expiratory airflow. The controller and power supply and module connector would not be in contact with any patient expired air or body fluids.

The removeable module in Figure 16a and 16b has three separate sensors.

Firstly, there is an SHT30 temperature and humidity sensor located on the support structure and placed in the airflow on the patient side, i.e. it is a downstream temperature sensor. The SHT temperature and humidity sensor is sufficiently removed from the other components to avoid interference in the readings, for example from heat emitted as part of the flow sensor operation.

Secondly, a flow sensor (Figure 16a item 9) comprises a resistive heater (Figure 16b item 4) with one thermistor located upstream (Figure 16b, item 5) and one thermistor located downstream (Figure 16b, item 3) on the central flow axis. The thermistors are each located on thin bridges with a narrow air gap between the bridges. The air gap, bridge spacing and bridge width is sufficient to avoid conductive and radiative heat transfer between the resistive heater element and the sensors. Comparison of the two sensor readings indicates the change of flow direction as the downstream thermistor will read convective heat from the heating resistor when it is downstream relative to the flow direction.

Thirdly, a thermistor acting as the moisture exchanger temperature sensor (Figure 16b, item 6) is located on the moisture exchanger side of the support structure, in contact with the moisture exchanger but with an air gap between the main sensor body and the support structure to maximise the thermistor's response to changes in the moisture exchanger temperature. The thermistor is located 1-2cm axially from the flow sensor heating resistor to avoid significant temperature reading interference.

The removeable module in Figure 16a also has a heating element, of the same design as was described with reference to Figure 9.

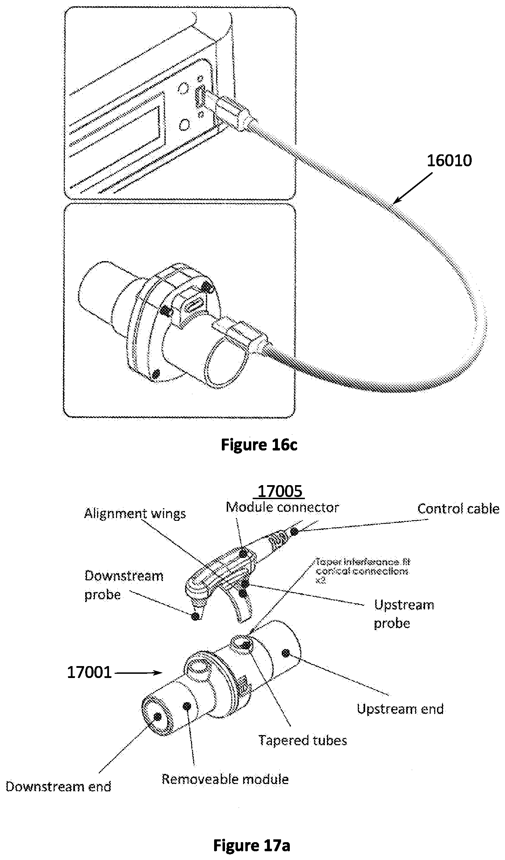

This sensor and heater combination includes a connection point for a module connector, in this case a reversible connector incorporated into the module. Figure 16c shows a cable 16010 including the module connector which connects the removeable module to the controller.

### Sensors in module connector

Figure 17a shows an example of a removeable module 17001 and module connector 17005 in which the module connector includes two conical temperature probes (sensors). The temperature probes and module connector are sealed so that they may be dipped into a sterilising fluid or sterilised in a steam environment.

One probe is an upstream temperature sensor and one probe is a downstream temperature sensor. The removeable module includes two slightly tapered tubes to accept the two probes, and has an upstream end and a downstream end. The module connector includes alignment wings shaped in arcs to partially envelope the outside of the removeable module. Due to being closer to one probe than the other, and also tapering to a smaller diameter on the upstream side of each arc, the wings make it impossible to attach the removeable module incorrectly. The control cable connects to the non-patient side of the module connector and goes to a controller or power supply.

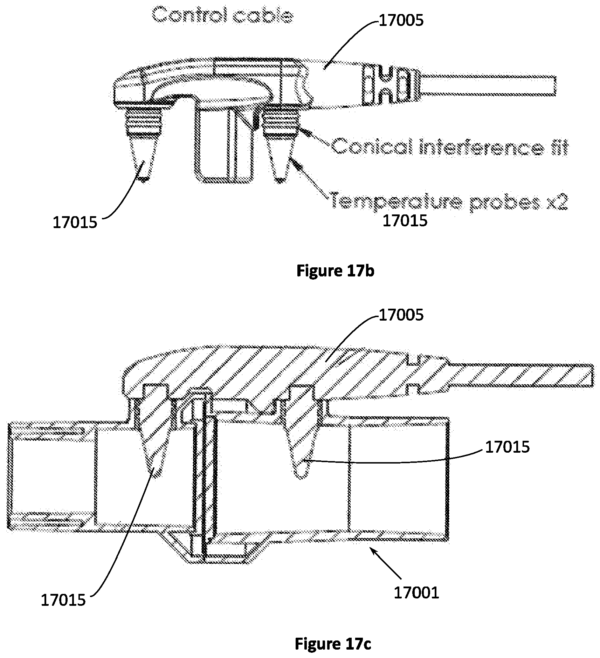

Figure 17b shows in detail that the probes 17015 have an upper substantially cylindrical region, with the conical temperature sensitive tip below.

Figure 17c shows how when the two parts in Figure 17a are connected, the probes pass into the removeable module via the tapered tubes and are presented into the airflow. The upper substantially cylindrical regions make an interference fit with the tapered tubes of the two temperature probes, holding the two components of the respiratory humidifying device firmly together despite airflow and pressure inside the removeable module.

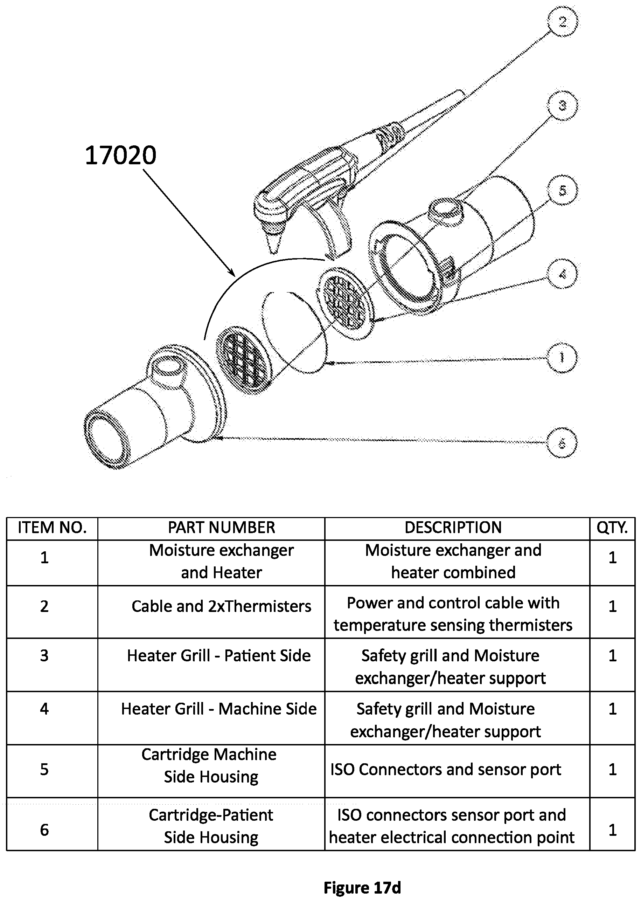

Figure 17d shows how the heater and moisture exchanger 17020 are sandwiched together between upstream and downstream parts of the removeable module, held in place by two substantially open grills which are held between the upstream and downstream parts. The upstream and downstream parts are permanently welded together and sealed during manufacturing, for example using ultrasonic welding. In some embodiments the grills may be formed as features of the upstream and downstream parts.

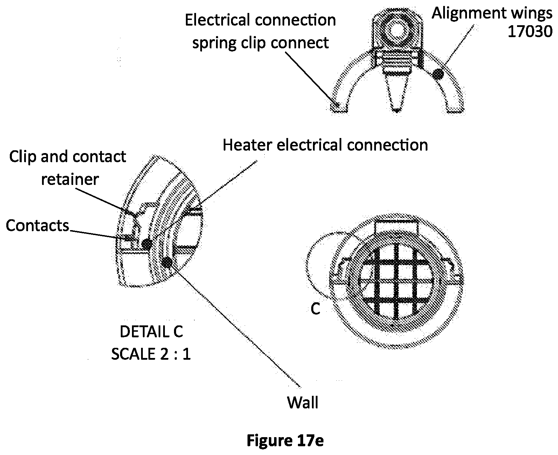

Figure 17e shows the alignment wings 17030 which partially envelope the removeable module when attached and keep the removeable module and module connector properly aligned. The alignment wings each include a spring clip connector on the inside, which is electrically connected to the control cable. The heater electrical connections are welded between the upstream and downstream parts and so pass through the opposite walls of the removeable module to contacts retained on the outside of the removeable module. The spring clip connectors on the wings make contact with the contacts when the removeable module and the module connector are properly attached. During attachment, the spring clip connectors pass over a retaining ridge on the outside of the removeable module, causing the removeable module and module connector to be positively locked together.

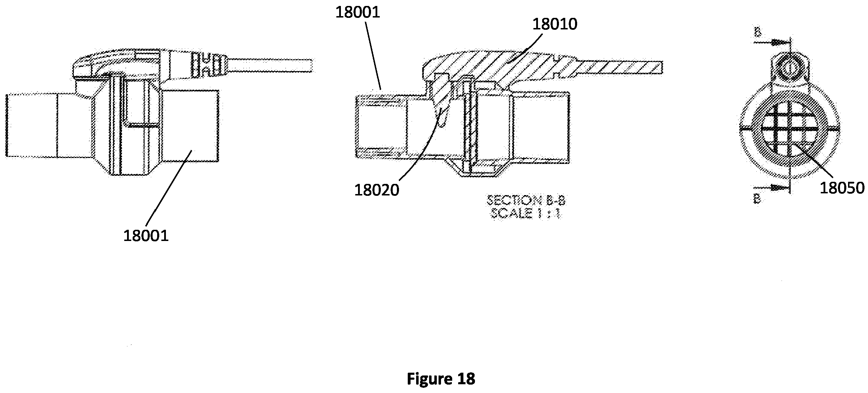

Figure 18 shows an example of a module 18001 with a single sensor probe 18020. The single probe sensor arrangement example is as per the dual probe arrangement in Figure 17, but with only one probe located on the patient side and a single corresponding tapered interference fit tube in the removeable module.

The removeable modules in Figure 17 and Figure 18 may have a bias flow vent and bacterial/viral filter incorporated into them.

### Removeable module electronics

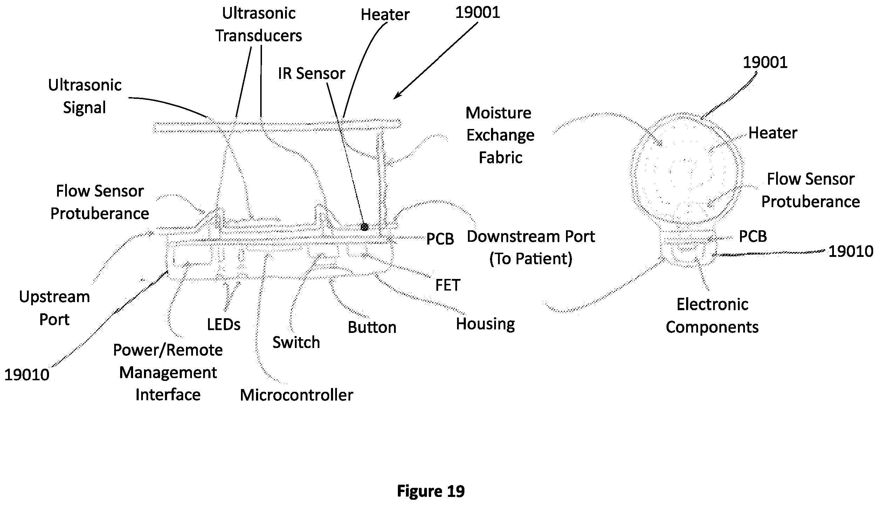

Figure 19 shows a simplified cross section of a removeable module 19001 and a module connector 19010 in which a controller is incorporated into the module connector. The controller comprises a PCB with a FET switch for controlling power to the heater, a button for receiving commands from a user, two LEDs for communicating status to the user, and an electrical connector for power and for communicating status and receiving commands from another device such as a remote user interface. A plastic housing is over-moulded over the module connector PCB and components but is thinned in places so that the LEDs can shine through to alert the user of any problems and the button can be pressed to select different modes of operation, such as invasive ventilation and non-invasive ventilation.

The controller also comprises an upstream and downstream ultrasonic transducer which, when the module connector is attached to the removeable module, fit into flow sensor protuberances in the wall of the removeable module and send an ultrasonic signal in alternating directions between them. In use, the controller uses the difference in time of flight of sound travelling upstream and downstream in the airflow, to determine when the patient is breathing in or breathing out. The protuberances protect the transducers from contact with the airflow.

Further, the heater includes contacts on the outer wall of the removeable module, which engage with contact pads on the PCB that protrude through the plastic housing.

Finally, an infra-red (IR) sensor receives light from the heater and moisture exchanger through the wall of the removeable module, and, once calibrated, allows the controller to measure the moisture exchanger and heater temperature. Alternatively, the resistance of the heating element could be measured to estimate the heater and moisture exchanger temperature.

Therefore, no part of the controller is in contact with the airflow, improving the hygiene of the respiratory humidifying device.

### Automatic mode selection

The user may select between one of several modes using the user interface, as described with reference to Figures 12, 13 or 14. In an alternative embodiment, more than one specification of removeable module may be manufactured, suitable for different uses such as invasive ventilation and non-invasive ventilation.

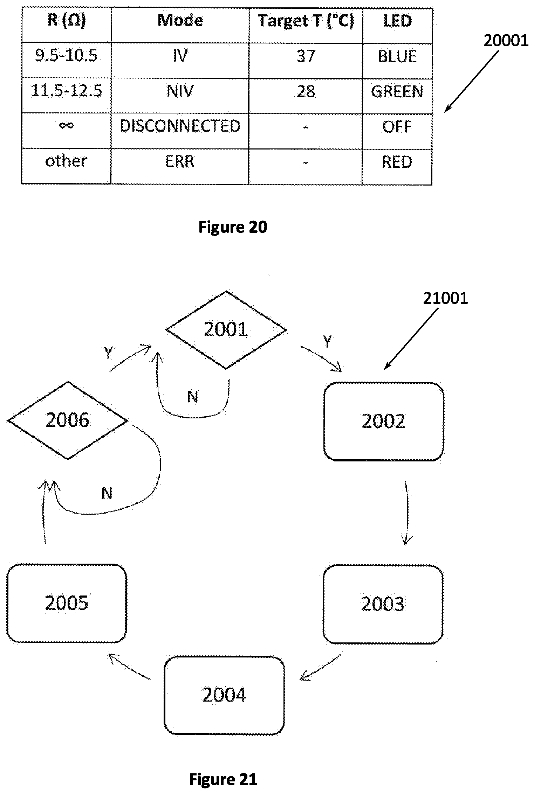

Figure 20 shows a selection table 20001 in the memory of an example controller. The controller measures the resistance of the heater in the removeable module, locates the corresponding row in the selection table, and thus determines which mode to operate in based on the selection table. The controller controls the temperature of the airflow during inspiration to match the Target T value, and also sets the colour of an LED indicator to the corresponding colour to indicate to the user the mode that has been selected.

Instead of measuring the resistance, alternative embodiments include the module connector having three contacts to connect with the heater element, and the controller detecting which type of removeable module has been connected based on the combination of contacts which connect to form a circuit. In yet another alterative, the controller may be able to measure the presence, absence or characteristic of a physical feature unique to each type of removeable module, such as a coloured patch on the removeable module.

In this way, the user does not have to change settings on a user interface to select the mode - they simply choose the labelled or colour-coded removeable module suitable for the intended application, and the respiratory humidifying device will configure itself appropriately. The LED indicator may, for example, match a colour printed on the removeable module, making it easy to establish that the respiratory humidifying device is configured correctly.

### Calibration

Due to manufacturing tolerances, each removeable module may be slightly different from the other. Therefore, it may be necessary to calibrate the controller to each individual removeable module.

Figure 21 shows an algorithm 21001 for calibrating the heater resistance, which may vary by for example +/-5% between modules. In step 2001, the controller determines if a connection has been made. If so, it measures the resistance of the heater (2002), calculates the ideal operating voltage according to a predetermined equation or look up table (2003), and configures the heater supply voltage accordingly (2004). It then begins to operate the respiratory humidifying device by turning on and off the heater (2005), until it detects that the removeable module has been disconnected (2006).

The controller similar calibration process may be required for any sensors in the removeable module.

### Applications

The respiratory humidifying device may be used with respiratory support devices where heated humidifiers, non-heated humidifiers and/or HMEs are used, for example anaesthetic delivery; tracheotomy; CPAP devices; Bi-level PAP devices; and home or hospital ventilators.

It may also be used in other applications where the patient is perfectly healthy, such as personal protection masks for general pollution, industrial pollution and for use in other dry environments, such as aircraft or desert or alpine settings, or any part of the world where people or animals experience dry air, especially cold dry air. In that case the system may have to operate off battery or non-mains power.

### Exemplary clinical setups

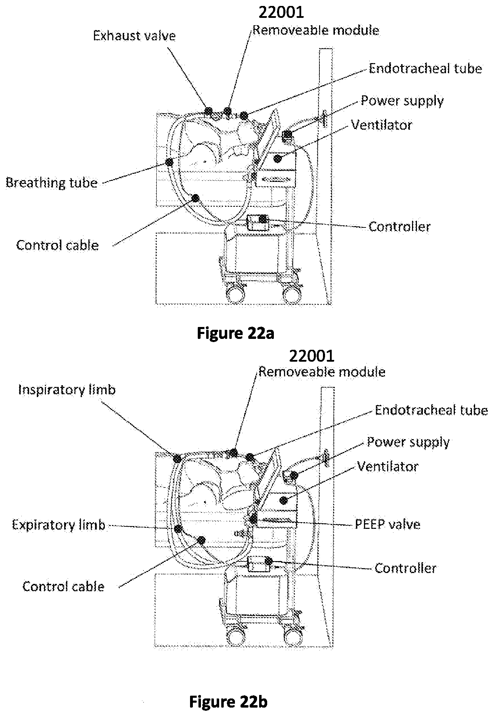

Figure 22a and Figure 22b show the respiratory humidifying device 22001 used in invasive ventilation with an endotracheal or tracheostomy tube. Both figures show the example embodiment provided in in Figure 16 and have a controller and power supply placed near the ventilator as suggested in Figure 10a. In Figure 22a the removeable module is connected to an exhaust valve, single limb breathing tube and ventilator. In Figure 22b the removeable module is connected to the inspiratory limb and expiratory limb of a dual limb breathing circuit, PEEP valve and ventilator.

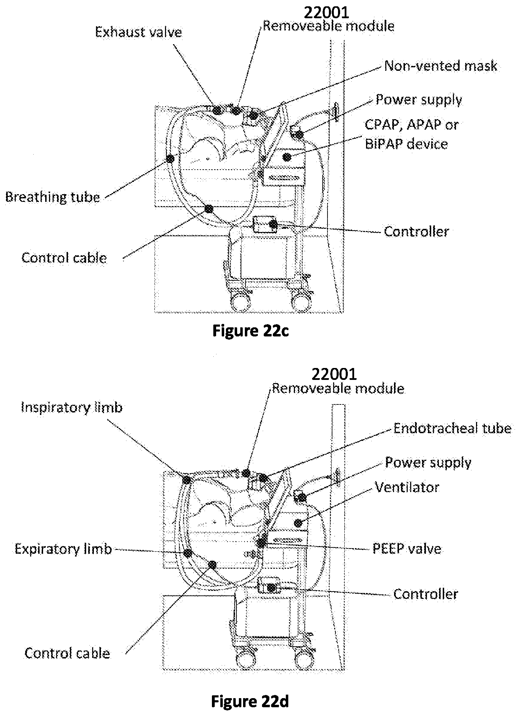

Figure 22c and Figure 22d show the respiratory humidifying device used in non-invasive ventilation with a non-vented mask. Both figures show the example embodiment provided in Figure 16 and have a controller and power supply near the ventilator as suggested in Figure 10a. In Figure 22c the removeable module is connected to an exhaust valve, single limb tube and CPAP, APAP or BiPAP respiratory support device. In Figure 22d the removeable module is connected to the inspiratory limb and expiratory limb of a dual limb breathing circuit, PEEP valve and ventilator.

### Combination of respiratory humidifying device and other devices

The respiratory humidifying device may be used in combination with a water humidifier in order to: provide higher humidification performance, different humidity profiles, use one to maximize the performance of the other, control rainout condensation in a patient interface and/or tubing, provide more accurate temperature control or provide more accurate humidity control.

The respiratory humidifying device may be used in combination with a liquid water reservoir to fill the moisture exchanger with liquid water droplets during inhale and release it during exhale by increasing the moisture exchanger temperature to 80°C approximately, to evaporate the liquid water.

### Controls

### Predictive power changes

As disclosed above, it is desirable for the controller to apply power to heat the moisture exchanger not only during the inspiration phase, but before the beginning of inspiration (and before any sensor(s) detect the beginning of inspiration); and to remove power (and heat) not only during the expiration phase but before the beginning of expiration (and before any sensor(s) detect the beginning of expiration) or at least provide the public with a useful choice.

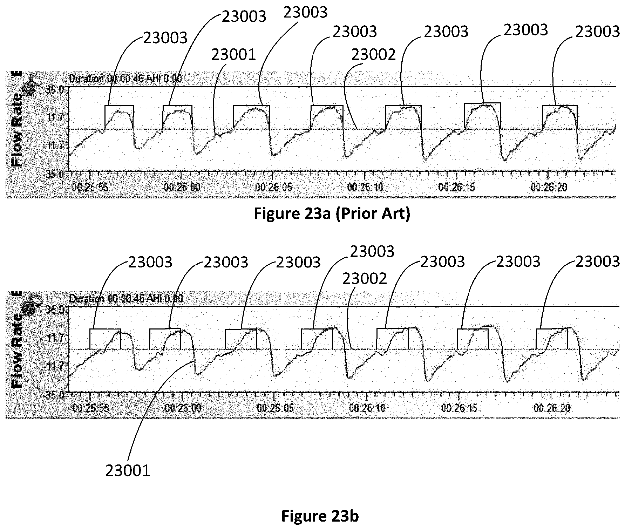

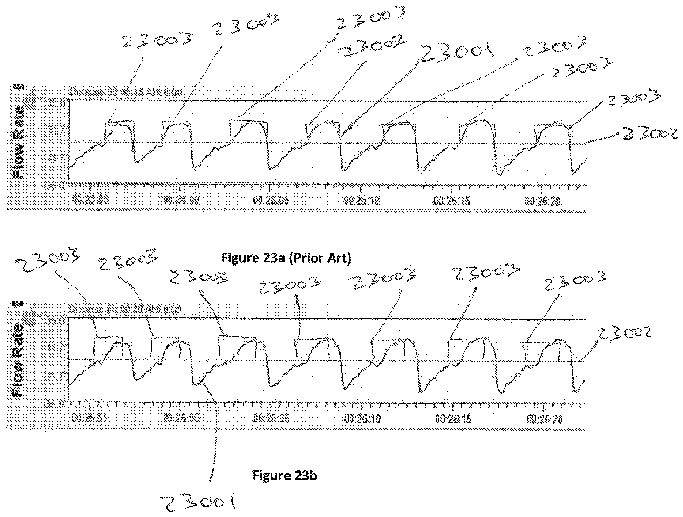

Figure 23a shows the behaviour of the prior art controller described in WO 2019/093910. When the black line 23001 is above the dashed line 23002 are the flow is inspiratory, and when the black line is below the dashed line the flow is expiratory. When the flow becomes inspiratory, the heater power (red lines) 23003 is switched on; when the flow become expiratory, the heater power is switched off.

Figure 23b shows how in one embodiment of the present invention the controller increases the heater power (switches the heater on) before the change in respiratory flow from expiration to inspiration and decreases the heater power (switches the heater off) before the change in respiratory flow from inspiration to expiration.

In this embodiment, the controller decreases the heater power (switches the heater off) before the change in respiratory flow from inspiration to expiration. Preferably this is done such that the moisture exchanger temperature falls to or below a second target temperature before expiration starts or at least before expiration has reached a significant portion of peak flow or total volume, for example 5%, 10%, 20% or even perhaps 30% thereof. Preferably the second target temperature is the LCST, or even 0.5°C or 1°C below the LCST.

Preferably this is done such that the moisture exchanger temperature rises to or above the first target temperature before inspiration starts or at least before inspiration has reached a significant portion of peak flow or total volume, for example 5%, 10%, 20% or even perhaps 30% thereof. Preferably also the controller controls (limits) the heater power (or keeps the heater off until close to the beginning of inspiration) such that the moisture exchanger temperature does not reach or exceed an undesired temperature before the end of expiration or before the majority of expiratory volume has passed the moisture exchanger (preferably 70% or 80% or even more preferably 90% thereof) or before the expiratory flow has significantly fallen (preferably below 30% or 20%, or even more preferably below 10% of peak expiratory flow). Preferably the desired or undesired temperature is the LCST, or more preferably they are at least 0.5°C or 1°C above and below the LCST respectively.

### Detecting flow

The controller may detect the magnitude or direction of the respiratory airflow using any of the known flow sensors available to the market.

### Distinctive points to control timing

In order to determine when to change the heater power, the controller may sense a measured parameter relating to the airflow through the moisture exchanger and uses the measured parameter to detect one or more distinctive points in the respiratory cycle. Preferably the measured parameter is the air flow rate and direction but in some alternative embodiments it may be the airflow pressure for example.

Ideally the one or more distinctive points include the change from inspiration to expiration and/or vice versa, or equivalently the beginning/end of expiration or inspiration. The beginning and/or end of inspiration and expiration may be detected by calculating when the measured parameter transitions from above to below a threshold value or from below to above the threshold value.

When the measured parameter is the airflow, the threshold value may be zero and, especially if the airflow sensor provides a direction, the beginning or end of expiration may be easy to detect. However, many cost effective sensors do not provide the airflow or the airflow direction, and even if the sensor does provide the airflow and direction the wide variety of ambient conditions the respiratory humidifying device has to operate in, and in particular the possibility of a patient interface leak error condition, means that in some embodiments the threshold value is determined by calculating a mean value of the measured parameter over an immediately preceding period of time which encompasses a plurality of respiratory cycles, for example 30s or 60s. The threshold value may then be the mean value of the measured parameter or it may be set a pre-determined amount above to below the mean value, or a proportional amount above or below the mean value compared to the maximum or minimum value of the measured parameter in the immediately preceding period of time which encompasses a plurality of respiratory cycles. By way of a specific example, the measured parameter may be the airflow (including its direction) through the moisture exchanger and the predictive controller may calculate the mean value of the airflow, detecting the approximate beginning of inspiration when the airflow goes above the mean of the airflow and the beginning of expiration when the airflow goes below the mean of the airflow.

In other embodiments the one or more distinctive points may be determined by the rate of change of the measured parameter being above or below a threshold value.

### Using time offsets from distinctive points

In order to achieve the desirable feature of changing the heater power before the beginning of inspiration or expiration, the controller may be a predictive controller which monitors successive inspiration or expiration cycles or distinctive points to learn the typical period of those cycles, or the period of the respiratory cycle as a whole. With knowledge of those periods, the predictive controller may change the heater power a time offset after one of more of the distinctive points in the respiratory cycle, with the expectation that it will be a known amount before the subsequent one.

In a preferred embodiment the time offset is proportional to the respiratory cycle period. The respiratory cycle period may be calculated from the mean of the elapsed time between one or more of the previous distinctive points.

### Plurality of time offsets

Further, there may be a plurality of time offsets relating to different distinctive points in a respiratory cycle and/or different changes in the power supply.

The plurality of distinctive points may be the beginning of inspiration and the beginning of expiration, so that the plurality of time offsets comprise an inspiration time offset and an expiration time offset. The inspiration time offset is the time offset after the change in respiratory flow from expiration to inspiration. In one embodiment the controller decreases the heater power (or switches the heater off) at the time which is the inspiration time offset after the change in respiratory flow from expiration to inspiration. The expiration time offset is the time offset after the change in respiratory flow from inspiration to expiration. The controller increases the heater power (or switches the heater on) at the time which is the expiration time offset after the change in respiratory flow from inspiration to expiration.

The time offset, inspiration time offset or the expiration time offset are preferably less than the time period of inspiration or the time period of expiration, or less than the sum of the time period of inspiration or time period of expiration. They are preferably less than 75% of, and more preferably less than 50% of, and even more preferably less than 33% of the time period of inspiration or time period of expiration.

Options for the minimum time offset are, for example 1s, 1.5s, or between 1s and 3s.

### Static time offset

In one embodiment the time offset may be pre-determined based on one or more of the time constant of the heater, the transfer rate of heat into the moisture exchanger, and the moisture exchanger response rate to temperature changes and the rate of absorption or desorption of moisture from the moisture exchanger.

### Dynamic time offset

The time offset may be a dynamic time offset that varies over time. The dynamic time offset may be determined using one or more of the respiratory cycle period, the desired or actual temperature and humidity of the inspiratory airflow, the humidity, temperature or flow rate of the expiratory airflow, and the temperature or humidity of the ambient air or supplied airflow.

In some embodiments an increase in the desired temperature or humidity of the inspiratory airflow leads to the dynamic time offset becoming smaller, i.e. leads to the controller changing the power supplied to the heating element earlier. Conversely, a decrease in the desired temperature or humidity of the inspiratory airflow leads to the dynamic time offset becoming larger, i.e. leads to the controller changing the power supplied to the heating element later.

### Phase-locked Loop to control timing

Another way of achieving the desirable feature of changing the heater power before the beginning of inspiration or expiration, is to use a Phase- and Frequency-locked Loop (PLL) which is synchronised to the respiratory cycle and driven by the measured parameter. The phase output of the PLL is used to determine whether to turn the heater on or off. The controller may turn the heater on and off at pre-determined or calculated phases with respect to the (PLL estimation of) respiratory cycle. The controller may change the target temperature at pre-determined or calculated phases with respect to the (PLL estimation of) respiratory cycle.

### Flow thresholds to control timing

Alternatively, the controller may apply heat when the airflow rate falls below a threshold. For example, when the expiratory airflow rate drops below 40% of the peak expiratory flow, or mean flow, heating may be applied. This percentage is an example, and in practice other flow rate thresholds may be apply, such as 20%, 30% or 50%. This allows time for the heat source to provide heat and to heat the moisture exchanger. This is a useful method as the end of the expiration cycle tends to be a relatively low flow rate over an extended period.

An alternative to this could be a tidal volume threshold, where a sensor, for example a flow sensor, is used to calculate the typical tidal volume and the heating is triggered when the tidal volume of the expiration reaches, for example 80% of the average tidal volume. This percentage is an example, and in practice other tidal volume thresholds may be apply, such as between, 40-80%, 80-90% or 90-99%.

The same triggering can be applied to the inspiration cycle, where the heating is stopped after inspiration flow drops below a threshold or tidal volume inspired exceeds a certain threshold. For example, when the inspiration flow rate drops below 40% of the peak flow, or mean flow, or the tidal volume inspired exceeds 80% of the expected tidal volume, heating may be stopped. This percentage is an example, and in practice other flow rate (or tidal volume) thresholds may be apply, such as between 20%, 30% or 50% (or 40-80%, 80-90% or 90-99%). This allows time for the heat source to provide heat and to heat the moisture exchanger.

The controller may actively adjust any of these thresholds to achieve the optimal moisture exchanger temperature profile, and this may vary from patient to patient depending on their tidal volume, breathing frequency, a setting controlled by a trained operator, and/or the wave form of their breathing. The controller may have a feedback loop, to control the timing of heating to reach the desired timing of moisture exchanger temperature, air temperature and/or humidity delivery in the breath cycle.

The threshold may also be adjusted to achieve a desired level of humidity. For example, if less humidity is required the cooling of the moisture exchanger on expiration may be delayed to reduce moisture build up in the moisture exchanger. Alternatively heating of the moisture exchanger on inspiration may be delayed. This may also be varied depending on the ambient temperature and humidity.

The above thresholds may be actively adjusted using feedback loops or may be pre-set values or be obtained from look up tables. Alternatively, they may be user controlled via an interface that increases or decreases the desired level of humidification.

### Heating control

### Constant heating time

The controller may apply heat to or remove heat from the moisture exchanger for a fixed period which does not (directly) depend on the respiratory cycle period. For example, the controller may apply heat for 1s or 2s, or most preferably 1.5s. In this way the controller can ensure that the all the moisture is released from the moisture exchanger, that a predictable temperature is reached, and that the moisture exchanger begins to cool at the earliest possible time once all the moisture is released from the moisture exchanger.

### Peak and hold

Preferably the controller of the respiratory humidifying device provides a peak of power at the beginning of its on stage to rapidly bring the element and moisture exchanger up to temperature, then reduces its power after a short period of time or once a certain temperature (resistance) is reached or calculated or expected to be reached.

Preferably there are a plurality of changes in power supplied to the heating element within a respiratory cycle which include a high power phase followed by a low power phase followed by a no power phase.

Preferably the high power phase is 100% of the maximum power that can be supplied by the controller, or at least 75%.

Preferably the low power phase is <70%, <50%, or less than 30%, of the maximum power that can be supplied by the controller.

Preferably the low power phase lasts for at least 2x as long, or at least 3x as long, or at least 4x as long as the high power phase.

Preferably the high power phase and low power phase together last for less than half the respiratory cycle period, or more preferably less than 40% of the respiratory cycle period.

Alternatively, the delivered power may be controlled in relation to the flow rate, or proportional to the flow rate, at any stage in the breathing cycle.

Alternatively, the delivered power may be controlled by an open loop feedforward or closed loop feedback controller (or a combination thereof) to maintain a desired moisture exchanger temperature or air temperature for inspiration, including different temperatures at different points during inspiration or expiration.

Preferably the high power phase and the low power phase are controlled such that they maintain the moisture exchanger at the desired moisture exchanger temperature for at least 100ms, or more preferably at least 200ms, during inspiration to allow time for the moisture exchanger to release moisture into the inspiratory airflow. The desired moisture exchanger temperature is preferably at or above the LCST. The times discussed depend on the application of the humidifying device, if used in a CPAP device the times are shorted, but if used on a tracheotomy tube or ventilator the times will be longer, up to 2 seconds probably. The power (%) provided may also adjust.

### Feedback control

In another embodiment the controller controls the temperature of the moisture exchanger using feedback from a moisture exchanger temperature sensor. The controller could use a PID controller or bang-bang (thermostat) controller.

### Error Detection

The respiratory humidifying device/apparatus is preferably able to detect one or more error conditions. Error conditions include error of sensors (open circuit, short circuit, invalid signal, loss of signal etc.); error of the heater/device (open circuit, short circuit, incorrect impedance); contamination of the heater or moisture exchanger; power supply out of allowable range; missing or incorrect moisture exchanger component or removeable module; ambient air conditions (temperature, humidity, pressure) out of correct operating range; error to replace removeable modules or other replaceable components in time (for example because their use has exceeded a safe amount since installation); breathing problems such as the patient interface being removed, falling off or leaking; loss of or change in air supply; blockage on the moisture exchanger not allowing air passing through (e.g. high condensation, sputum, secretions).

These error conditions may lead to unnecessary power consumption, unsafe operation, inadequate or excessive therapeutic output (flow, humidity, temperature, pressure) or patient annoyance.

Therefore, upon detecting an error condition the respiratory humidifying device preferably alerts the user or patient through sounds (tones, noises, spoken messages), flashing light(s), interruption of or restriction of the air flow, or communicating with the patient, users, healthcare professionals or a remote management device or another part of the breathing circuit. The respiratory humidifying device may cease operation in some error conditions.

The respiratory humidifying device may also alert the user or patient through any of the above means during episodes of sleep apnoea, respiratory distress, difficulty breathing etc., or when their frequency or number exceeds a threshold.

### Detecting leaks

A critical error condition in respiratory assistive devices is leakage of air from the patient interface, around the seal between the patient and the interface. This is particularly true of the respiratory humidifying device of the present invention, because the device generally needs to capture as much of the expired moisture as possible to provide the maximum humidity. Therefore, it is desirable to have a method of detecting patient interface leak error conditions.

It is a further aspect of the present disclosure to provide a method of detecting patient interface leak error conditions in a respiratory humidifying device according to other aspects of the disclosure, the method comprising monitoring at least one sensor value associated with the air flow through the respiratory humidifying device and comparing it to a set of expected sensor values, and detecting an error condition when the at least one sensor value is not within the set of expected sensor values. The method is preferably performed at least every 10 respiratory cycles, and preferably every cycle.

The at least one sensor value may be a rate of change or a sensor value.

A patient interface leak error condition may be detected by any of the methods disclosed in the prior art, for example measuring the average or peak flow rates through the respiratory humidifying device. However, the leak detection according to the above description may be advantageous as it is carried out closer to the patient and may be more sensitive, or more easily able to distinguish between leaks at the patient interface or leaks at another point in the air supply upstream from the moisture exchanger.

### Illustration only

While the present technology has been illustrated by the description of the embodiments thereof, and while the embodiments have been described in detail, it is not the intention of the Applicant to restrict or in any way limit the scope of the appended claims to such detail. Additional advantages and modifications will readily appear to those skilled in the art. Therefore, the invention in its broader aspects is not limited to the specific details, representative apparatus, and illustrative examples shown and described. Accordingly, departures may be made from such details without departure from the scope of the invention, which is defined by the following appended claims.

## Claims

1. A respiratory humidifying device (2001, 2010, 3001, 6001, 7001) for use in a respiratory humidifying system having an inspiration and expiration respiratory cycle, the respiratory humidifying device (2001, 2010, 3001, 6001, 7001) comprising:
a moisture exchanger (4001, 9005, 17020) positioned such that airflow occurring in the inspiration and expiration respiratory cycle is in contact with the moisture exchanger (4001, 9005, 17020), the moisture exchanger (4001, 9005, 17020) being temperature-responsive and having a critical solution temperature;
a heater(9010); and
a controller controlling a supply of power to the heater (9010)such that:
during expiration the moisture exchanger (4001, 9005, 17020) extracts moisture from the airflow; and
during inspiration the moisture exchanger (4001, 9005, 17020) returns moisture to the airflow,
**characterised in that**:
the moisture exchanger (4001, 9005, 17020) and the heater (9010) are contained in a removeable module (16001, 17001, 18001, 19001) and wherein the removeable module (16001, 17001, 18001, 19001) comprises a connector which impermanently engages with a module connector configured to pass electrical signals between the controller and the removable module (16001, 17001, 18001, 19001);
the removeable module (16001, 17001, 18001, 19001) comprises an open upstream end for making a detachable connection for airflow from a respiratory support device or an upstream breathing circuit connected to the respiratory support device;
and the removeable module (16001, 17001, 18001, 19001) comprises an open downstream end for making a detachable connection for airflow to and from a patient interface or a downstream breathing circuit connected to the patient interface;
the module connector comprises the controller.

2. The respiratory humidifying device (2001, 2010, 3001, 6001, 7001) of claim 1 further comprising at least one sensor for measuring a parameter of the airflow, wherein at least one of the at least one sensor is selected from the group consisting of a temperature sensor, an airflow speed sensor, an airflow direction sensor, a pressure sensor, a CO2 sensor, a O2 sensor, a CO sensor and a humidity sensor, and wherein the controller supplies power to the heater (9010) in response to the measured parameter of the airflow.

3. The respiratory humidifying device (2001, 2010, 3001, 6001, 7001) of claim 1 or claim 2 wherein the only part of the respiratory humidifying device (2001, 2010, 3001, 6001, 7001) in contact with the airflow is the removeable module (16001, 17001, 18001, 19001) and the parts contained therein.

4. The respiratory humidifying device (2001, 2010, 3001, 6001, 7001) of any one of claims 1 to 3 wherein the removeable module (16001, 17001, 18001, 19001) comprises at least one removeable module sensor.

5. The respiratory humidifying device (2001, 2010, 3001, 6001, 7001) of claim 4 wherein at least one of the at least one removeable module sensor measures a parameter of the airflow or the moisture exchanger (4001, 9005, 17020) temperature without being in contact with the airflow.

6. The respiratory humidifying device (2001, 2010, 3001, 6001, 7001) of claim 4 wherein at least one of the at least one removeable module sensor measures a parameter of the airflow or the moisture exchanger (4001, 9005, 17020) temperature through a wall of the removeable module (16001, 17001, 18001, 19001).

7. The respiratory humidifying device (2001, 2010, 3001, 6001, 7001) of any one of claims 4 to 6 wherein at least one of the at least one removeable module sensor is selected from the group consisting of an ultrasonic airflow speed, a direction sensor and an infrared temperature sensor.

8. The respiratory humidifying device (2001, 2010, 3001, 6001, 7001) any one of claims 1 to 7 wherein the module connector comprises at least one module connector sensor.

9. The respiratory humidifying device (2001, 2010, 3001, 6001, 7001) of claim 8 wherein at least one of the at least one module connector sensor measures a parameter of the airflow or the moisture exchanger (4001, 9005, 17020) temperature without being in contact with the airflow.

10. The respiratory humidifying device (2001, 2010, 3001, 6001, 7001) of any one of claims 1 to 9 wherein the module connector comprises a user interface.

11. The respiratory humidifying device (2001, 2010, 3001, 6001, 7001) of any one of claims 1 to 10 wherein the controller includes a mode selector for selecting one of a plurality of operating modes, each of the operating modes providing different amounts of, or timing of, power to the heater (9010), in response to a measured airflow.

12. The respiratory humidifying device (2001, 2010, 3001, 6001, 7001) of claim 11 wherein the mode selector comprises module sensing means to measure a physical characteristic of the removeable module (16001, 17001, 18001, 19001), wherein a selection table of physical characteristics of a plurality of different removeable module types and desired operation modes of the plurality of different removeable module types is stored in the memory of the controller, and the mode selector selects from the selection table the operating mode that best matches the measured physical characteristic.

13. The respiratory humidifying device (2001, 2010, 3001, 6001, 7001) of any one of claims 1 to 12 wherein the controller comprises calibration means to measure a physical characteristic of the removeable module (16001, 17001, 18001, 19001), and the controller is operable to detect connection of a new removeable module and to use the calibration means to measure the physical characteristic of the new removeable module.

14. The respiratory humidifying device (2001, 2010, 3001, 6001, 7001) of any one of claims 1 to 13 wherein the heater (9010) is in contact with the moisture exchanger (4001, 9005, 17020) and in contact with the airflow.

15. The respiratory humidifying device (2001, 2010, 3001, 6001, 7001) of claim 14 including a second heater not in contact with the moisture exchanger (4001, 9005, 17020) and in contact with the airflow.

## Patentansprüche

1. Atemluftbefeuchtungsgerät (2001, 2010, 3001, 6001, 7001) zur Verwendung in einem Atemluftbefeuchtungssystem mit einem Inspirations- und Exspirations-Atemzyklus, wobei das Atemluftbefeuchtungsgerät (2001, 2010, 3001, 6001, 7001) umfasst:
einen Feuchtigkeitsaustauscher (4001, 9005, 17020), der so positioniert ist, dass der im Inspirations- und Exspirations-Atemzyklus auftretende Luftstrom in Kontakt mit dem Feuchtigkeitsaustauscher (4001, 9005, 17020) steht, wobei der Feuchtigkeitsaustauscher (4001, 9005, 17020) temperaturabhängig ist und eine kritische Lösungstemperatur aufweist,
ein Heizgerät (9010); und
ein Steuergerät, das eine Stromversorgung des Heizgeräts (9010) so steuert, dass:
während des Exspirations der Feuchtigkeitsaustauscher (4001, 9005, 17020) Feuchtigkeit aus dem Luftstrom entzieht; und
während des Inspirations der Feuchtigkeitsaustauscher (4001, 9005, 17020) Feuchtigkeit an den Luftstrom zurückgibt,
**dadurch gekennzeichnet, dass**:
der Feuchtigkeitsaustauscher (4001, 9005, 17020) und das Heizgerät (9010) in einem herausnehmbaren Modul (16001, 17001, 18001, 19001) enthalten sind und wobei das herausnehmbare Modul (16001, 17001, 18001, 19001) einen Verbinder umfasst, der unbeständig in einen Modulverbinder eingreift, der so beschaffen ist, dass er elektrische Signale zwischen dem Steuergerät und dem herausnehmbaren Modul (16001, 17001, 18001, 19001) weiterleitet;
das abnehmbare Modul (16001, 17001, 18001, 19001) ein offenes stromaufwärts gelegenes Ende aufweist, um eine lösbare Verbindung für den Luftstrom von einem Atemunterstützungsgerät oder einem stromaufwärts gelegenen Atemzyklus herzustellen, der mit dem Atemunterstützungsgerät verbunden ist;
und das abnehmbare Modul (16001, 17001, 18001, 19001) ein offenes stromabwärts gelegenes Ende aufweist, um eine lösbare Verbindung für den Luftstrom zu und von einer Patientenschnittstelle oder einem stromabwärts gelegenen, mit der Patientenschnittstelle verbundenen Atemzyklus herzustellen;
der Modulverbinder das Steuergerät umfasst.

2. Atemluftbefeuchtungsgerät (2001, 2010, 3001, 6001, 7001) nach Anspruch 1 ferner umfassend mindestens einen Sensor zum Messen eines Parameters des Luftstroms, wobei mindestens einer des mindestens einen Sensors ausgewählt ist aus der Gruppe bestehend aus einem Temperatursensor, einem Luftstromgeschwindigkeitssensor, einem Luftstromrichtungssensor, einem Drucksensor, einem CO2-Sensor, einem O2-Sensor, einem CO-Sensor und einem Feuchtigkeitssensor ausgewählt ist, und wobei das Steuergerät das Heizgerät (9010) in Reaktion auf den gemessenen Parameter des Luftstroms mit Strom versorgt.

3. Atemluftbefeuchtungsgerät (2001, 2010, 3001, 6001, 7001) nach Anspruch 1 oder Anspruch 2, wobei der einzige Teil des Atemluftbefeuchtungsgeräts (2001, 2010, 3001, 6001, 7001), der mit dem Luftstrom in Kontakt ist, das herausnehmbare Modul (16001, 17001, 18001, 19001) und die darin enthaltenen Teile sind.

4. Atemluftbefeuchtungsgerät (2001, 2010, 3001, 6001, 7001) nach einem der Ansprüche 1 bis 3, wobei das herausnehmbare Modul (16001, 17001, 18001, 19001) mindestens einen herausnehmbaren Modulsensor umfasst.

5. Atemluftbefeuchtungsgerät (2001, 2010, 3001, 6001, 7001) nach Anspruch 4, wobei mindestens einer des mindestens eines herausnehmbaren Modulsensors einen Parameter des Luftstroms oder die Temperatur des Feuchtigkeitstauschers (4001, 9005, 17020) misst, ohne mit dem Luftstrom in Kontakt zu sein.

6. Atemluftbefeuchtungsgerät (2001, 2010, 3001, 6001, 7001) nach Anspruch 4, wobei mindestens einer des mindestens eines herausnehmbaren Modulsensors einen Parameter des Luftstroms oder die Temperatur des Feuchtigkeitstauschers (4001, 9005, 17020) durch eine Wand des herausnehmbaren Moduls (16001, 17001, 18001, 19001) misst.

7. Atemluftbefeuchtungsgerät (2001, 2010, 3001, 6001, 7001) nach einem der Ansprüche 4 bis 6, wobei mindestens einer des mindestens eines herausnehmbaren Modulsensors aus der Gruppe ausgewählt ist, die aus einem Ultraschall-Luftstromssensor, einem Richtungssensor und einem Infrarot-Temperatursensor besteht.

8. Atemluftbefeuchtungsgerät (2001, 2010, 3001, 6001, 7001) nach einem der Ansprüche 1 bis 7, wobei der Modulverbinder mindestens einen Modulverbindersensor umfasst.

9. Atemluftbefeuchtungsgerät (2001, 2010, 3001, 6001, 7001) nach Anspruch 8, wobei mindestens einer des mindestens eines herausnehmbaren Modulsensors einen Parameter des Luftstroms oder die Temperatur des Feuchtigkeitstauschers (4001, 9005, 17020) misst, ohne mit dem Luftstrom in Kontakt zu sein.

10. Atemluftbefeuchtungsgerät (2001, 2010, 3001, 6001, 7001) nach einem der Ansprüche 1 bis 9, wobei der Modulverbinder eine Benutzerschnittstelle umfasst.

11. Atemluftbefeuchtungsgerät (2001, 2010, 3001, 6001, 7001) nach einem der Ansprüche 1 bis 10, wobei das Steuergerät einen Betriebsartenwähler zur Auswahl einer von mehreren Betriebsarten umfasst, wobei jede der Betriebsarten als Reaktion auf einen gemessenen Luftstrom unterschiedliche Leistungsmengen oder -zeitsteuerung für das Heizgerät (9010) bereitstellt.

12. Atemluftbefeuchtungsgerät (2001, 2010, 3001, 6001, 7001) nach Anspruch 11, wobei der Betriebsartenwähler die Modulsensormittel umfasst, um eine physikalische Eigenschaft des herausnehmbaren Moduls (16001, 17001, 18001, 19001) zu messen, wobei eine Auswahltabelle physikalischer Eigenschaften einer Vielzahl verschiedener herausnehmbarer Modultypen und gewünschter Betriebsarten der Vielzahl verschiedener herausnehmbarer Modultypen im Speicher des Steuergeräts gespeichert ist und der Betriebsartenwähler aus der Auswahltabelle die Betriebsart auswählt, die der gemessenen physikalischen Eigenschaft am besten entspricht.

13. Atemluftbefeuchtungsgerät (2001, 2010, 3001, 6001, 7001) nach einem der Ansprüche 1 bis 12, wobei das Steuergerät Kalibriermittel umfasst, um eine physikalische Eigenschaft des herausnehmbaren Moduls (16001, 17001, 18001, 19001) zu messen, und das Steuergerät so betrieben werden kann, dass es den Verbindungsanschluss eines neuen herausnehmbaren Moduls erkennt und die Kalibriermittel verwendet, um die physikalische Eigenschaft des neuen herausnehmbaren Moduls zu messen.

14. Atemluftbefeuchtungsgerät (2001, 2010, 3001, 6001, 7001) nach einem der Ansprüche 1 bis 13, wobei das Heizgerät (9010) in Kontakt mit dem Feuchtigkeitsaustauscher (4001, 9005, 17020) und in Kontakt mit dem Luftstrom steht.

15. Atemluftbefeuchtungsgerät (2001, 2010, 3001, 6001, 7001) nach Anspruch 14 mit einem zweiten Heizgerät, das nicht in Kontakt mit dem Feuchtigkeitsaustauscher (4001, 9005, 17020) und in Kontakt mit dem Luftstrom steht.

## Revendications

1. Dispositif d'humidification respiratoire (2001, 2010, 3001, 6001, 7001) destiné à être utilisé dans un système d'humidification respiratoire ayant un cycle respiratoire d'inspiration et d'expiration, le dispositif d'humidification respiratoire (2001, 2010, 3001, 6001, 7001) comprenant :
un échangeur d'humidité (4001, 9005, 17020) positionné de telle sorte que le flux d'air se produisant dans le cycle respiratoire d'inspiration et d'expiration est en contact avec l'échangeur d'humidité (4001, 9005, 17020), l'échangeur d'humidité (4001, 9005, 17020) réagissant à la température et ayant une température de solution critique ;
un dispositif de chauffage (9010) ; et
un contrôleur qui commande l'alimentation électrique du dispositif de chauffage (9010) de telle sorte que :
pendant l'expiration, l'échangeur d'humidité (4001, 9005, 17020) extrait l'humidité du flux d'air ; et
pendant l'inspiration, l'échangeur d'humidité (4001, 9005, 17020) restitue l'humidité au flux d'air,
**caractérisé par le fait que** :
l'échangeur d'humidité (4001, 9005, 17020) et le dispositif de chauffage (9010) sont contenus dans un module amovible (16001, 17001, 18001, 19001) et dans lequel le module amovible (16001, 17001, 18001, 19001) comprend un connecteur qui s'engage de manière impermanente avec un connecteur de module configuré pour transmettre des signaux électriques entre le contrôleur et le module amovible (16001, 17001, 18001, 19001) ;
le module amovible (16001, 17001, 18001, 19001) comprend une extrémité amont ouverte pour établir une connexion détachable pour le flux d'air provenant d'un dispositif d'assistance respiratoire ou d'un circuit respiratoire en amont connecté à l'dispositif d'assistance respiratoire ;
et le module amovible (16001, 17001, 18001, 19001) comprend une extrémité aval ouverte pour établir une connexion amovible pour le flux d'air vers et depuis une interface patient ou un circuit respiratoire en aval connecté à l'interface patient ;
le connecteur de module comprend le contrôleur.

2. Dispositif d'humidification respiratoire (2001, 2010, 3001, 6001, 7001) de la revendication 1 comprend en outre au moins un capteur pour mesurer un paramètre du flux d'air, dans lequel au moins un de l'au moins un capteur est sélectionné dans le groupe constitué d'un capteur de température, un capteur de vitesse du flux d'air, un capteur de direction du flux d'air, un capteur de pression, un capteur de CO2, un capteur d'O2, un capteur de CO et un capteur d'humidité, et dans lequel le contrôleur alimente le dispositif de chauffage (9010) en électricité en réponse au paramètre mesuré du flux d'air.

3. Dispositif d'humidification respiratoire (2001, 2010, 3001, 6001, 7001) de la revendication 1 ou de la revendication 2 dans lequel la seule partie du dispositif d'humidification respiratoire (2001, 2010, 3001, 6001, 7001) en contact avec le flux d'air est le module amovible (16001, 17001, 18001, 19001) et les pièces qu'il contient.

4. Dispositif d'humidification respiratoire (2001, 2010, 3001, 6001, 7001) de l'une quelconque des revendications 1 à 3, dans lequel le module amovible (16001, 17001, 18001, 19001) comprend au moins un capteur de module amovible.

5. Dispositif d'humidification respiratoire (2001, 2010, 3001, 6001, 7001) de la revendication 4 dans lequel au moins un de l'au moins un capteur de module amovible mesure un paramètre du flux d'air ou la température de l'échangeur d'humidité (4001, 9005, 17020) sans être en contact avec le flux d'air.

6. Dispositif d'humidification respiratoire (2001, 2010, 3001, 6001, 7001) de la revendication 4 dans lequel au moins un de l'au moins un capteur du module amovible mesure un paramètre du flux d'air ou la température de l'échangeur d'humidité (4001, 9005, 17020) à travers une paroi du module amovible (16001, 17001, 18001, 19001).

7. Dispositif d'humidification respiratoire (2001, 2010, 3001, 6001, 7001) de l'une quelconque des revendications 4 à 6 dans lequel au moins de l'au moins un de capteur du module amovible est sélectionné dans le groupe constitué d'un capteur de vitesse d'flux d'air à ultrasons, d'un capteur de direction et d'un capteur de température à infrarouge.

8. Dispositif d'humidification respiratoire (2001, 2010, 3001, 6001, 7001) de l'une quelconque des revendications 1 à 7, dans lequel le connecteur de module comprend au moins un capteur de connecteur de module.

9. Dispositif d'humidification respiratoire (2001, 2010, 3001, 6001, 7001) de la revendication 8 dans lequel au moins un de l'au moins un capteur de connecteur de module mesure un paramètre du flux d'air ou la température de l'échangeur d'humidité (4001, 9005, 17020) sans être en contact avec le flux d'air.

10. Dispositif d'humidification respiratoire (2001, 2010, 3001, 6001, 7001) de l'une quelconque des revendications 1 à 9 dans lequel le connecteur de module comprend une interface utilisateur.

11. Dispositif d'humidification respiratoire (2001, 2010, 3001, 6001, 7001) de l'une quelconque des revendications 1 à 10 dans lequel le contrôleur comprend un sélecteur de mode pour sélectionner l'un de plusieurs modes de fonctionnement, chacun des modes de fonctionnement fournissant des quantités différentes, ou une synchronisation différente, de l'alimentation électrique au dispositif de chauffage (9010), en réponse à un flux d'air mesuré.

12. Dispositif d'humidification respiratoire (2001, 2010, 3001, 6001, 7001) de la revendication 11 dans lequel le sélecteur de mode comprend des moyens de détection de module pour mesurer une caractéristique physique du module amovible (16001, 17001, 18001, 19001), dans lequel un tableau de sélection des caractéristiques physiques d'une pluralité de différents types de modules amovibles et des modes de fonctionnement souhaités de la pluralité de différents types de modules amovibles est stocké dans la mémoire du contrôleur, et le sélecteur de mode sélectionne à partir du tableau de sélection le mode de fonctionnement qui correspond le mieux à la caractéristique physique mesurée.

13. Dispositif d'humidification respiratoire (2001, 2010, 3001, 6001, 7001) de l'une quelconque des revendications 1 à 12 dans lequel le contrôleur comprend des moyens d'étalonnage pour mesurer une caractéristique physique du module amovible (16001, 17001, 18001, 19001) et le contrôleur est actionnable pour détecter la connexion d'un nouveau module amovible et d'utiliser les moyens d'étalonnage pour mesurer la caractéristique physique du nouveau module amovible.

14. Dispositif d'humidification respiratoire (2001, 2010, 3001, 6001, 7001) de l'une quelconque des revendications 1 à 13 dans lequel le dispositif de chauffage (9010) est en contact avec l'échangeur d'humidité (4001, 9005, 17020) et en contact avec le flux d'air.

15. Dispositif d'humidification respiratoire (2001, 2010, 3001, 6001, 7001) de la revendication 14 comprenant un second dispositif de chauffage qui n'est pas en contact avec l'échangeur d'humidité (4001, 9005, 17020) et en contact avec le flux d'air.
